(19) Europäisches Patentamt / European Patent Office / Office européen des brevets

(11) **EP 3 733 838 A1**

(12) **EUROPEAN PATENT APPLICATION**
published in accordance with Art. 153(4) EPC

(43) Date of publication:
04.11.2020 Bulletin 2020/45

(51) Int Cl.:
*C12N 5/0775* $^{(2010.01)}$    *A61K 35/28* $^{(2015.01)}$
*A61K 35/50* $^{(2015.01)}$    *A61K 35/51* $^{(2015.01)}$
*A61P 1/04* $^{(2006.01)}$    *A61P 3/10* $^{(2006.01)}$
*A61P 17/06* $^{(2006.01)}$    *A61P 19/02* $^{(2006.01)}$
*A61P 25/00* $^{(2006.01)}$    *A61P 29/00* $^{(2006.01)}$
*A61P 37/02* $^{(2006.01)}$    *A61P 37/06* $^{(2006.01)}$
*C12N 15/09* $^{(2006.01)}$    *C12Q 1/6844* $^{(2018.01)}$

(21) Application number: 18897164.2

(22) Date of filing: 28.12.2018

(86) International application number:
PCT/JP2018/048539

(87) International publication number:
WO 2019/132025 (04.07.2019 Gazette 2019/27)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 28.12.2017 JP 2017253878

(71) Applicant: Kaneka Corporation
Osaka-shi, Osaka 530-8288 (JP)

(72) Inventors:
• **INO Keita**
  **Kobe-shi, Hyogo 650-0047 (JP)**
• **KOBAYASHI Chiho**
  **Kobe-shi, Hyogo 650-0047 (JP)**
• **YAMAGUCHI Sho**
  **Kobe-shi, Hyogo 650-0047 (JP)**
• **UMEDA Nobuyoshi**
  **Kobe-shi, Hyogo 650-0047 (JP)**

(74) Representative: Hoffmann Eitle
Patent- und Rechtsanwälte PartmbB
Arabellastraße 30
81925 München (DE)

(54) **CELL POPULATION INCLUDING ADHESIVE STEM CELLS, METHOD FOR PRODUCING SUCH CELL POPULATION, AND PHARMACEUTICAL COMPOSITION**

(57)    An object of the present invention is to provide a cell population comprising adherent stem cells and exhibiting high immunosuppressive activity and/or proliferative property and a production method of the cell population, and a pharmaceutical composition comprising the cell population. The present invention provides a production method of a cell population comprising adherent stem cells, the method comprising obtaining a cell population having the following cell characteristics (a) and (b): (a) the cell population is positive for expressions of PLIN2 gene and NEFM gene, (b) the cell population satisfies the relative expression level of PLIN2 gene to the expression level of SDHA gene of 1.50 or more.

SEQUENCE LISTING
<110> kaneka corporation
<120> CELL POPULATION THAT INCLUDES MESENCHYMAL STEM CELLS, METHOD FOR
PRODUCING THE SAME, AND PHARMACEUTICAL COMPOSITION
<130> 181521A
<160> 14
<170> PatentIn version 3.5
<210> 1
<211> 1314
<212> DNA
<213> Homo sapiens
<220>
<221> CDS
<222> (1)..(1314)
<400> 1

```
atg gca tcc gtt gca gtt gat cca caa ccg agt gtg gtg act cgg gtg        48
Met Ala Ser Val Ala Val Asp Pro Gln Pro Ser Val Val Thr Arg Val
1               5                   10                  15
gtc aac ctg ccc ttg gtg agc tcc acg tat gac ctc atg tcc tca gcc        96
Val Asn Leu Pro Leu Val Ser Ser Thr Tyr Asp Leu Met Ser Ser Ala
                20                  25                  30
tat ctc agt aca aag gac cag tat ccc tac ctg aag tct gtg tgt gag       144
Tyr Leu Ser Thr Lys Asp Gln Tyr Pro Tyr Leu Lys Ser Val Cys Glu
            35                  40                  45
atg gca gag aac ggt gtg aag acc atc acc tcc gtg gcc atg acc agt       192
Met Ala Glu Asn Gly Val Lys Thr Ile Thr Ser Val Ala Met Thr Ser
        50                  55                  60
gct ctg ccc atc atc cag aag cta gag ccg caa att gca gtt gcc aat       240
Ala Leu Pro Ile Ile Gln Lys Leu Glu Pro Gln Ile Ala Val Ala Asn
65                  70                  75                  80
acc tat gcc tgt aag ggg cta gac agg att gag gag aga ctg cct att       288
Thr Tyr Ala Cys Lys Gly Leu Asp Arg Ile Glu Glu Arg Leu Pro Ile
                85                  90                  95
ctg aat cag cca tca act cag att gtt gcc aat gcc aaa ggc gct gtg       336
Leu Asn Gln Pro Ser Thr Gln Ile Val Ala Asn Ala Lys Gly Ala Val
                100                 105                 110
act ggg gca aaa gat gct gtg acg act act gtg act ggg gcc aag gat       384
Thr Gly Ala Lys Asp Ala Val Thr Thr Thr Val Thr Gly Ala Lys Asp
            115                 120                 125
tct gtg gcc agc acg atc aca ggg gtg atg gac aag acc aaa ggg gca       432
Ser Val Ala Ser Thr Ile Thr Gly Val Met Asp Lys Thr Lys Gly Ala
        130                 135                 140
gtg act ggc agt gtg gag aag acc aag tct gtg gtc agt ggc agc att       480
Val Thr Gly Ser Val Glu Lys Thr Lys Ser Val Val Ser Gly Ser Ile
145                 150                 155                 160
aac aca gtc ttg ggg agt cgg atg atg cag ctc gtg agc agt ggc gta       528
Asn Thr Val Leu Gly Ser Arg Met Met Gln Leu Val Ser Ser Gly Val
                165                 170                 175
gaa aat gca ctc acc aaa tca gag ctg ttg gta gaa cag tac ctc cct       576
Glu Asn Ala Leu Thr Lys Ser Glu Leu Leu Val Glu Gln Tyr Leu Pro
                180                 185                 190
ctc act gag gaa gaa cta gaa aaa gaa gca aaa aaa gtt gaa gga ttt       624
Leu Thr Glu Glu Glu Leu Glu Lys Glu Ala Lys Lys Val Glu Gly Phe
            195                 200                 205
gat ctg gtt cag aag cca agt tat tat gtt aga ctg gga tcc ctg tct       672
Asp Leu Val Gln Lys Pro Ser Tyr Tyr Val Arg Leu Gly Ser Leu Ser
        210                 215                 220
acc aag ctt cac tcc cgt gcc tac cag cag gct ctc agc agg gtt aaa       720
Thr Lys Leu His Ser Arg Ala Tyr Gln Gln Ala Leu Ser Arg Val Lys
225                 230                 235                 240
gaa gct aag caa aaa agc caa cag acc att tct cag ctc cat tct act       768
Glu Ala Lys Gln Lys Ser Gln Gln Thr Ile Ser Gln Leu His Ser Thr
                245                 250                 255
```

```
gtt cac ctg att gaa ttt gcc agg aag aat gtg tat agt gcc aat cag        816
Val His Leu Ile Glu Phe Ala Arg Lys Asn Val Tyr Ser Ala Asn Gln
            260                 265                 270
aaa att cag gat gct cag gat aag ctc tac ctc tca tgg gta gag tgg        864
Lys Ile Gln Asp Ala Gln Asp Lys Leu Tyr Leu Ser Trp Val Glu Trp
        275                 280                 285
aaa agg agc att gga tat gat gat act gat gag tcc cac tgt gct gag        912
Lys Arg Ser Ile Gly Tyr Asp Asp Thr Asp Glu Ser His Cys Ala Glu
    290                 295                 300
cac att gag tca cgt act ctt gca att gcc cgc aac ctg act cag cag        960
His Ile Glu Ser Arg Thr Leu Ala Ile Ala Arg Asn Leu Thr Gln Gln
305                 310                 315                 320
ctc cag acc acg tgc cac acc ctc ctg tcc aac atc caa ggt gta cca       1008
Leu Gln Thr Thr Cys His Thr Leu Leu Ser Asn Ile Gln Gly Val Pro
                325                 330                 335
cag aac atc caa gat caa gcc aag cac atg ggg gtg atg gca ggc gac       1056
Gln Asn Ile Gln Asp Gln Ala Lys His Met Gly Val Met Ala Gly Asp
            340                 345                 350
atc tac tca gtg ttc cgc aat gct gcc tcc ttt aaa gaa gtg tct gac       1104
Ile Tyr Ser Val Phe Arg Asn Ala Ala Ser Phe Lys Glu Val Ser Asp
        355                 360                 365
agc ctc ctc act tct agc aag ggg cag ctg cag aaa atg aag gaa tct       1152
Ser Leu Leu Thr Ser Ser Lys Gly Gln Leu Gln Lys Met Lys Glu Ser
    370                 375                 380
tta gat gac gtg atg gat tat ctt gtt aac aac acg ccc ctc aac tgg       1200
Leu Asp Asp Val Met Asp Tyr Leu Val Asn Asn Thr Pro Leu Asn Trp
385                 390                 395                 400
ctg gta ggt ccc ttt tat cct cag ctg act gag tct cag aat gct cag       1248
Leu Val Gly Pro Phe Tyr Pro Gln Leu Thr Glu Ser Gln Asn Ala Gln
                405                 410                 415
gac caa ggt gca gag atg gac aag agc agc cag gag acc cag cga tct       1296
Asp Gln Gly Ala Glu Met Asp Lys Ser Ser Gln Glu Thr Gln Arg Ser
            420                 425                 430
gag cat aaa act cat taa                                               1314
Glu His Lys Thr His
            435

<210>   2
<211>   437
<212>   PRT
<213>   Homo sapiens
<400>   2
Met Ala Ser Val Ala Val Asp Pro Gln Pro Ser Val Val Thr Arg Val
1               5                   10                  15
Val Asn Leu Pro Leu Val Ser Ser Thr Tyr Asp Leu Met Ser Ser Ala
            20                  25                  30
Tyr Leu Ser Thr Lys Asp Gln Tyr Pro Tyr Leu Lys Ser Val Cys Glu
        35                  40                  45
Met Ala Glu Asn Gly Val Lys Thr Ile Thr Ser Val Ala Met Thr Ser
    50                  55                  60
Ala Leu Pro Ile Ile Gln Lys Leu Glu Pro Gln Ile Ala Val Ala Asn
65                  70                  75                  80
Thr Tyr Ala Cys Lys Gly Leu Asp Arg Ile Glu Glu Arg Leu Pro Ile
                85                  90                  95
Leu Asn Gln Pro Ser Thr Gln Ile Val Ala Asn Ala Lys Gly Ala Val
                100                 105                 110
Thr Gly Ala Lys Asp Ala Val Thr Thr Thr Val Thr Gly Ala Lys Asp
            115                 120                 125
Ser Val Ala Ser Thr Ile Thr Gly Val Met Asp Lys Thr Lys Gly Ala
    130                 135                 140
Val Thr Gly Ser Val Glu Lys Thr Lys Ser Val Val Ser Gly Ser Ile
145                 150                 155                 160
Asn Thr Val Leu Gly Ser Arg Met Met Gln Leu Val Ser Ser Gly Val
                165                 170                 175
```

```
Glu Asn Ala Leu Thr Lys Ser Glu Leu Leu Val Glu Gln Tyr Leu Pro
            180                 185                 190
Leu Thr Glu Glu Glu Leu Glu Lys Glu Ala Lys Lys Val Glu Gly Phe
            195                 200                 205
Asp Leu Val Gln Lys Pro Ser Tyr Tyr Val Arg Leu Gly Ser Leu Ser
        210                 215                 220
Thr Lys Leu His Ser Arg Ala Tyr Gln Gln Ala Leu Ser Arg Val Lys
225                 230                 235                 240
Glu Ala Lys Gln Lys Ser Gln Gln Thr Ile Ser Gln Leu His Ser Thr
                245                 250                 255
Val His Leu Ile Glu Phe Ala Arg Lys Asn Val Tyr Ser Ala Asn Gln
            260                 265                 270
Lys Ile Gln Asp Ala Gln Asp Lys Leu Tyr Leu Ser Trp Val Glu Trp
            275                 280                 285
Lys Arg Ser Ile Gly Tyr Asp Asp Thr Asp Glu Ser His Cys Ala Glu
            290                 295                 300
His Ile Glu Ser Arg Thr Leu Ala Ile Ala Arg Asn Leu Thr Gln Gln
305                 310                 315                 320
Leu Gln Thr Thr Cys His Thr Leu Leu Ser Asn Ile Gln Gly Val Pro
                325                 330                 335
Gln Asn Ile Gln Asp Gln Ala Lys His Met Gly Val Met Ala Gly Asp
            340                 345                 350
Ile Tyr Ser Val Phe Arg Asn Ala Ala Ser Phe Lys Glu Val Ser Asp
            355                 360                 365
Ser Leu Leu Thr Ser Ser Lys Gly Gln Leu Gln Lys Met Lys Glu Ser
        370                 375                 380
Leu Asp Asp Val Met Asp Tyr Leu Val Asn Asn Thr Pro Leu Asn Trp
385                 390                 395                 400
Leu Val Gly Pro Phe Tyr Pro Gln Leu Thr Glu Ser Gln Asn Ala Gln
            405                 410                 415
Asp Gln Gly Ala Glu Met Asp Lys Ser Ser Gln Glu Thr Gln Arg Ser
            420                 425                 430
Glu His Lys Thr His
            435
```

<210> 3
<211> 1623
<212> DNA
<213> Homo sapiens
<220>
<221> CDS
<222> (1)..(1623)
<400> 3

```
atg gct cgt cat ttg cgc gaa tac cag gac ctc ctc aac gtc aag atg      48
Met Ala Arg His Leu Arg Glu Tyr Gln Asp Leu Leu Asn Val Lys Met
1               5                   10                  15
gct ctg gat ata gaa atc gct gcg tac aga aaa ctc ctg gag ggt gaa      96
Ala Leu Asp Ile Glu Ile Ala Ala Tyr Arg Lys Leu Leu Glu Gly Glu
            20                  25                  30
gag act aga ttt agc aca ttt gca gga agc atc act ggg cca ctg tat     144
Glu Thr Arg Phe Ser Thr Phe Ala Gly Ser Ile Thr Gly Pro Leu Tyr
        35                  40                  45
aca cac cga ccc cca atc aca ata tcc agt aag att cag aaa ccc aag     192
Thr His Arg Pro Pro Ile Thr Ile Ser Ser Lys Ile Gln Lys Pro Lys
        50                  55                  60
gtg gaa gct ccc aag ctt aag gtc caa cac aaa ttt gtc gag gag atc     240
Val Glu Ala Pro Lys Leu Lys Val Gln His Lys Phe Val Glu Glu Ile
65                  70                  75                  80
ata gag gaa acc aaa gtg gag gat gag aag tca gaa atg gaa gag gcc     288
Ile Glu Glu Thr Lys Val Glu Asp Glu Lys Ser Glu Met Glu Glu Ala
                85                  90                  95
ctg aca gcc att aca gag gaa ttg gcc gtt tcc atg aag gaa gag aag     336
Leu Thr Ala Ile Thr Glu Glu Leu Ala Val Ser Met Lys Glu Glu Lys
            100                 105                 110
```

```
aaa gaa gca gca gaa gaa aag gaa gag gaa ccc gaa gct gaa gaa gaa        384
Lys Glu Ala Ala Glu Glu Lys Glu Glu Glu Pro Glu Ala Glu Glu Glu
        115             120             125

gaa gta gct gcc aaa aag tct cca gtg aaa gca act gca cct gaa gtt        432
Glu Val Ala Ala Lys Lys Ser Pro Val Lys Ala Thr Ala Pro Glu Val
    130             135             140

aaa gaa gag gaa ggg gaa aag gag gaa gaa gaa ggc cag gaa gaa gag        480
Lys Glu Glu Glu Gly Glu Lys Glu Glu Glu Glu Gly Gln Glu Glu Glu
145             150             155             160

gag gaa gaa gat gag gga gct aag tca gac caa gcc gaa gag gga gga        528
Glu Glu Glu Asp Glu Gly Ala Lys Ser Asp Gln Ala Glu Glu Gly Gly
            165             170             175

tcc gag aag gaa ggc tct agt gaa aaa gag gaa ggt gag cag gaa gaa        576
Ser Glu Lys Glu Gly Ser Ser Glu Lys Glu Glu Gly Glu Gln Glu Glu
            180             185             190

gga gaa aca gaa gct gaa gct gaa gga gag gaa gcc gaa gct aaa gag        624
Gly Glu Thr Glu Ala Glu Ala Glu Gly Glu Glu Ala Glu Ala Lys Glu
            195             200             205

gaa aag aaa gtg gag gaa aag agt gag gaa gtg gct acc aag gag gag        672
Glu Lys Lys Val Glu Glu Lys Ser Glu Glu Val Ala Thr Lys Glu Glu
    210             215             220

ctg gtg gca gat gcc aag gtg gaa aag cca gaa aaa gcc aag tct cct        720
Leu Val Ala Asp Ala Lys Val Glu Lys Pro Glu Lys Ala Lys Ser Pro
225             230             235             240

gtg cca aaa tca cca gtg gaa gag aaa ggc aag tct cct gtg ccc aag        768
Val Pro Lys Ser Pro Val Glu Glu Lys Gly Lys Ser Pro Val Pro Lys
            245             250             255

tca cca gtg gaa gag aaa ggc aag tct cct gtg ccc aag tca cca gtg        816
Ser Pro Val Glu Glu Lys Gly Lys Ser Pro Val Pro Lys Ser Pro Val
            260             265             270

gaa gag aaa ggc aag tct cct gtg ccg aaa tca cca gtg gaa gag aaa        864
Glu Glu Lys Gly Lys Ser Pro Val Pro Lys Ser Pro Val Glu Glu Lys
            275             280             285

ggc aag tct cct gtg tca aaa tca cca gtg gaa gag aaa gcc aaa tct        912
Gly Lys Ser Pro Val Ser Lys Ser Pro Val Glu Glu Lys Ala Lys Ser
            290             295             300

cct gtg cca aaa tca cca gtg gaa gag gca aag tca aaa gca gaa gtg        960
Pro Val Pro Lys Ser Pro Val Glu Glu Ala Lys Ser Lys Ala Glu Val
305             310             315             320

ggg aaa ggt gaa cag aaa gag gaa gaa gaa aag gaa gtc aag gaa gct       1008
Gly Lys Gly Glu Gln Lys Glu Glu Glu Glu Lys Glu Val Lys Glu Ala
            325             330             335

ccc aag gaa gag aag gta gag aaa aag gaa gag aaa cca aag gat gtg       1056
Pro Lys Glu Glu Lys Val Glu Lys Lys Glu Glu Lys Pro Lys Asp Val
            340             345             350

cca gag aag aag aaa gct gag tcc cct gta aag gag gaa gct gtg gca       1104
Pro Glu Lys Lys Lys Ala Glu Ser Pro Val Lys Glu Glu Ala Val Ala
            355             360             365

gag gtg gtc acc atc acc aaa tcg gta aag gtg cac ttg gag aaa gag       1152
Glu Val Val Thr Ile Thr Lys Ser Val Lys Val His Leu Glu Lys Glu
            370             375             380

acc aaa gaa gag ggg aag cca ctg cag cag gag aaa gag aag gag aaa       1200
Thr Lys Glu Glu Gly Lys Pro Leu Gln Gln Glu Lys Glu Lys Glu Lys
385             390             395             400

gcg gga gga gag gga gga agt gag gag gaa ggg agt gat aaa ggt gcc       1248
Ala Gly Gly Glu Gly Gly Ser Glu Glu Glu Gly Ser Asp Lys Gly Ala
            405             410             415

aag gga tcc agg aag gaa gac ata gct gtc aat ggg gag gta gaa gga       1296
Lys Gly Ser Arg Lys Glu Asp Ile Ala Val Asn Gly Glu Val Glu Gly
            420             425             430

aaa gag gag gta gag cag gag acc aag gaa aaa ggc agt ggg agg gaa       1344
Lys Glu Glu Val Glu Gln Glu Thr Lys Glu Lys Gly Ser Gly Arg Glu
            435             440             445
```

```
gag gag aaa ggc gtt gtc acc aat ggc cta gac ttg agc cca gca gat      1392
Glu Glu Lys Gly Val Val Thr Asn Gly Leu Asp Leu Ser Pro Ala Asp
    450                     455                 460
gaa aag aag ggg ggt gat aaa agt gag gag aaa gtg gtg gtg acc aaa      1440
Glu Lys Lys Gly Gly Asp Lys Ser Glu Glu Lys Val Val Val Thr Lys
465                     470                 475                 480
acg gta gaa aaa atc acc agt gag ggg gga gat ggt gct acc aaa tac      1488
Thr Val Glu Lys Ile Thr Ser Glu Gly Gly Asp Gly Ala Thr Lys Tyr
                485                 490                 495
atc act aaa tct gta acc gtc act caa aag gtt gaa gag cat gaa gag      1536
Ile Thr Lys Ser Val Thr Val Thr Gln Lys Val Glu Glu His Glu Glu
                500                 505                 510
acc ttt gag gag aaa cta gtg tct act aaa aag gta gaa aaa gtc act      1584
Thr Phe Glu Glu Lys Leu Val Ser Thr Lys Lys Val Glu Lys Val Thr
            515                 520                 525
tca cac gcc ata gta aag gaa gtc acc cag agt gac taa                  1623
Ser His Ala Ile Val Lys Glu Val Thr Gln Ser Asp
            530                 535                 540
```

<210> 4
<211> 540
<212> PRT
<213> Homo sapiens

<400> 4

```
Met Ala Arg His Leu Arg Glu Tyr Gln Asp Leu Leu Asn Val Lys Met
1               5                   10                  15
Ala Leu Asp Ile Glu Ile Ala Ala Tyr Arg Lys Leu Leu Glu Gly Glu
                20                  25                  30
Glu Thr Arg Phe Ser Thr Phe Ala Gly Ser Ile Thr Gly Pro Leu Tyr
            35                  40                  45
Thr His Arg Pro Pro Ile Thr Ile Ser Ser Lys Ile Gln Lys Pro Lys
        50                  55                  60
Val Glu Ala Pro Lys Leu Lys Val Gln His Lys Phe Val Glu Glu Ile
65                  70                  75                  80
Ile Glu Glu Thr Lys Val Glu Asp Glu Lys Ser Glu Met Glu Glu Ala
                85                  90                  95
Leu Thr Ala Ile Thr Glu Glu Leu Ala Val Ser Met Lys Glu Glu Lys
            100                 105                 110
Lys Glu Ala Ala Glu Glu Lys Glu Glu Glu Pro Glu Ala Glu Glu Glu
        115                 120                 125
Glu Val Ala Ala Lys Lys Ser Pro Val Lys Ala Thr Ala Pro Glu Val
    130                 135                 140
Lys Glu Glu Glu Gly Glu Lys Glu Glu Glu Glu Gly Gln Glu Glu Glu
145                 150                 155                 160
Glu Glu Glu Asp Glu Gly Ala Lys Ser Asp Gln Ala Glu Glu Gly Gly
                165                 170                 175
Ser Glu Lys Glu Gly Ser Ser Glu Lys Glu Glu Gly Glu Gln Glu Glu
            180                 185                 190
Gly Glu Thr Glu Ala Glu Ala Glu Gly Glu Glu Ala Glu Ala Lys Glu
            195                 200                 205
Glu Lys Lys Val Glu Glu Lys Ser Glu Glu Val Ala Thr Lys Glu Glu
    210                 215                 220
Leu Val Ala Asp Ala Lys Val Glu Lys Pro Glu Lys Ala Lys Ser Pro
225                 230                 235                 240
Val Pro Lys Ser Pro Val Glu Glu Lys Gly Lys Ser Pro Val Pro Lys
                245                 250                 255
Ser Pro Val Glu Glu Lys Gly Lys Ser Pro Val Pro Lys Ser Pro Val
                260                 265                 270
Glu Glu Lys Gly Lys Ser Pro Val Pro Lys Ser Pro Val Glu Glu Lys
            275                 280                 285
Gly Lys Ser Pro Val Ser Lys Ser Pro Val Glu Glu Lys Ala Lys Ser
        290                 295                 300
Pro Val Pro Lys Ser Pro Val Glu Glu Ala Lys Ser Lys Ala Glu Val
305                 310                 315                 320
```

```
Gly Lys Gly Glu Gln Lys Glu Glu Glu Glu Lys Glu Val Lys Glu Ala
            325                 330             335
Pro Lys Glu Glu Lys Val Glu Lys Lys Glu Glu Lys Pro Lys Asp Val
            340                 345             350
Pro Glu Lys Lys Lys Ala Glu Ser Pro Val Lys Glu Glu Ala Val Ala
            355                 360             365
Glu Val Thr Ile Thr Lys Ser Val Lys Val His Leu Glu Lys Glu
    370                 375             380
Thr Lys Glu Glu Gly Lys Pro Leu Gln Gln Glu Lys Glu Lys Glu Lys
385                 390             395                 400
Ala Gly Gly Glu Gly Gly Ser Glu Glu Glu Gly Ser Asp Lys Gly Ala
                405                 410             415
Lys Gly Ser Arg Lys Glu Asp Ile Ala Val Asn Gly Glu Val Glu Gly
            420                 425             430
Lys Glu Glu Val Glu Gln Glu Thr Lys Glu Lys Gly Ser Gly Arg Glu
    435                 440             445
Glu Glu Lys Gly Val Val Thr Asn Gly Leu Asp Leu Ser Pro Ala Asp
    450                 455             460
Glu Lys Lys Gly Gly Asp Lys Ser Glu Glu Lys Val Val Val Thr Lys
465                 470                 475                 480
Thr Val Glu Lys Ile Thr Ser Glu Gly Gly Asp Gly Ala Thr Lys Tyr
            485                 490             495
Ile Thr Lys Ser Val Thr Val Thr Gln Lys Val Glu Glu His Glu Glu
            500                 505             510
Thr Phe Glu Glu Lys Leu Val Ser Thr Lys Lys Val Glu Lys Val Thr
        515                 520             525
Ser His Ala Ile Val Lys Glu Val Thr Gln Ser Asp
    530                 535             540
```

```
<210>  5
<211>  1851
<212>  DNA
<213>  Homo sapiens
<220>
<221>  CDS
<222>  (1)..(1851)
<400>  5
atg tcg ggg gtc cgg ggc ctg tcg cgg ctg ctg agc gct cgg cgc ctg      48
Met Ser Gly Val Arg Gly Leu Ser Arg Leu Leu Ser Ala Arg Arg Leu
1               5                   10                  15
gcg ctg gcc aag gcg tgg cca aca gtg ttg caa aca gga acc cga ggt      96
Ala Leu Ala Lys Ala Trp Pro Thr Val Leu Gln Thr Gly Thr Arg Gly
                20                  25                  30
ttt cac ttc act gtt gat ggg aac aag agg gca tct gct aaa gtt tca     144
Phe His Phe Thr Val Asp Gly Asn Lys Arg Ala Ser Ala Lys Val Ser
            35                  40                  45
gat tcc att tct gct cag tat cca gta gtg gat cat gaa ttt gat gca     192
Asp Ser Ile Ser Ala Gln Tyr Pro Val Val Asp His Glu Phe Asp Ala
        50                  55                  60
gtg gtg gta ggc gct gga ggg gca ggc ttg cga gct gca ttt ggc ctt     240
Val Val Val Gly Ala Gly Gly Ala Gly Leu Arg Ala Ala Phe Gly Leu
65                  70                  75                  80
tct gag gca ggg ttt aat aca gca tgt gtt acc aag ctg ttt cct acc     288
Ser Glu Ala Gly Phe Asn Thr Ala Cys Val Thr Lys Leu Phe Pro Thr
                85                  90                  95
agg tca cac act gtt gca gca cag cta gaa aat tat ggc atg ccg ttt     336
Arg Ser His Thr Val Ala Ala Gln Leu Glu Asn Tyr Gly Met Pro Phe
                100                 105                 110
agc aga act gaa gat ggg aag att tat cag cgt gca ttt ggt gga cag     384
Ser Arg Thr Glu Asp Gly Lys Ile Tyr Gln Arg Ala Phe Gly Gly Gln
            115                 120                 125
agc ctc aag ttt gga aag ggc ggg cag gcc cat cgg tgc tgc tgt gtg     432
Ser Leu Lys Phe Gly Lys Gly Gly Gln Ala His Arg Cys Cys Cys Val
        130                 135                 140
```

40

```
gct gat cgg act ggc cac tcg cta ttg cac acc tta tat gga agg tct        480
Ala Asp Arg Thr Gly His Ser Leu Leu His Thr Leu Tyr Gly Arg Ser
145             150                 155                 160
ctg cga tat gat acc agc tat ttt gtg gag tat ttt gcc ttg gat ctc        528
Leu Arg Tyr Asp Thr Ser Tyr Phe Val Glu Tyr Phe Ala Leu Asp Leu
                165                 170                 175
ctg atg gag aat ggg gag tgc cgt ggt gtc atc gca ctg tgc ata gag        576
Leu Met Glu Asn Gly Glu Cys Arg Gly Val Ile Ala Leu Cys Ile Glu
            180                 185                 190
gac ggg tcc atc cat cgc ata aga gca aag aac act gtt gtt gcc aca        624
Asp Gly Ser Ile His Arg Ile Arg Ala Lys Asn Thr Val Val Ala Thr
        195                 200                 205
gga ggc tac ggg cgc acc tac ttc agc tgc acg tct gcc cac acc agc        672
Gly Gly Tyr Gly Arg Thr Tyr Phe Ser Cys Thr Ser Ala His Thr Ser
210                 215                 220
act ggc gac ggc acg gcc atg atc acc agg gca ggc ctt cct tgc cag        720
Thr Gly Asp Gly Thr Ala Met Ile Thr Arg Ala Gly Leu Pro Cys Gln
225                 230                 235                 240
gac cta gag ttt gtt cag ttc cac cct aca ggc ata tat ggt gct ggt        768
Asp Leu Glu Phe Val Gln Phe His Pro Thr Gly Ile Tyr Gly Ala Gly
                245                 250                 255
tgt ctc att acg gaa gga tgt cgt gga gag gga ggc att ctc att aac        816
Cys Leu Ile Thr Glu Gly Cys Arg Gly Glu Gly Gly Ile Leu Ile Asn
            260                 265                 270
agt caa ggc gaa agg ttt atg gag cga tac gcc cct gtc gcg aag gac        864
Ser Gln Gly Glu Arg Phe Met Glu Arg Tyr Ala Pro Val Ala Lys Asp
        275                 280                 285
ctg gcg tct aga gat gtg gtg tct cgg tcc atg act ctg gag atc cga        912
Leu Ala Ser Arg Asp Val Val Ser Arg Ser Met Thr Leu Glu Ile Arg
    290                 295                 300
gaa gga aga ggc tgt ggc cct gag aaa gat cac gtc tac ctg cag ctg        960
Glu Gly Arg Gly Cys Gly Pro Glu Lys Asp His Val Tyr Leu Gln Leu
305                 310                 315                 320
cac cac cta cct cca gag cag ctg gcc acg cgc ctg cct ggc att tca       1008
His His Leu Pro Pro Glu Gln Leu Ala Thr Arg Leu Pro Gly Ile Ser
                325                 330                 335
gag aca gcc atg atc ttc gct ggc gtg gac gtc acg aag gag ccg atc       1056
Glu Thr Ala Met Ile Phe Ala Gly Val Asp Val Thr Lys Glu Pro Ile
            340                 345                 350
cct gtc ctc ccc acc gtg cat tat aac atg ggc ggc att ccc acc aac       1104
Pro Val Leu Pro Thr Val His Tyr Asn Met Gly Gly Ile Pro Thr Asn
        355                 360                 365
tac aag ggg cag gtc ctg agg cac gtg aat ggc cag gat cag att gtg       1152
Tyr Lys Gly Gln Val Leu Arg His Val Asn Gly Gln Asp Gln Ile Val
370                 375                 380
ccc ggc ctg tac gcc tgt ggg gag gcc gcc tgt gcc tcg gta cat ggt       1200
Pro Gly Leu Tyr Ala Cys Gly Glu Ala Ala Cys Ala Ser Val His Gly
385                 390                 395                 400
gcc aac cgc ctc ggg gca aac tcg ctc ttg gac ctg gtt gtc ttt ggt       1248
Ala Asn Arg Leu Gly Ala Asn Ser Leu Leu Asp Leu Val Val Phe Gly
                405                 410                 415
cgg gca tgt gcc ctg agc atc gaa gag tca tgc agg cct gga gat aaa       1296
Arg Ala Cys Ala Leu Ser Ile Glu Glu Ser Cys Arg Pro Gly Asp Lys
            420                 425                 430
gtc cct cca att aaa cca aac gct ggg gaa gaa tct gtc atg aat ctt       1344
Val Pro Pro Ile Lys Pro Asn Ala Gly Glu Glu Ser Val Met Asn Leu
        435                 440                 445
gac aaa ttg aga ttt gct gat gga agc ata aga aca tcg gaa ctg cga       1392
Asp Lys Leu Arg Phe Ala Asp Gly Ser Ile Arg Thr Ser Glu Leu Arg
    450                 455                 460
ctc agc atg cag aag tca atg caa aat cat gct gcc gtg ttc cgt gtg       1440
Leu Ser Met Gln Lys Ser Met Gln Asn His Ala Ala Val Phe Arg Val
465                 470                 475                 480
```

```
gga agc gtg ttg caa gaa ggt tgt ggg aaa atc agc aag ctc tat gga        1488
Gly Ser Val Leu Gln Glu Gly Cys Gly Lys Ile Ser Lys Leu Tyr Gly
            485                     490                 495
gac cta aag cac ctg aag acg ttc gac cgg gga atg gtc tgg aac acg        1536
Asp Leu Lys His Leu Lys Thr Phe Asp Arg Gly Met Val Trp Asn Thr
            500                     505                 510
gac ctg gtg gag acc ctg gag ctg cag aac ctg atg ctg tgt gcg ctg        1584
Asp Leu Val Glu Thr Leu Glu Leu Gln Asn Leu Met Leu Cys Ala Leu
            515                     520                 525
cag acc atc tac gga gca gag gca cgg aag gag tca cgg ggc gcg cat        1632
Gln Thr Ile Tyr Gly Ala Glu Ala Arg Lys Glu Ser Arg Gly Ala His
            530                     535                 540
gcc agg gaa gac tac aag gtg cgg att gat gag tac gat tac tcc aag        1680
Ala Arg Glu Asp Tyr Lys Val Arg Ile Asp Glu Tyr Asp Tyr Ser Lys
545                     550                     555                 560
ccc atc cag ggg caa cag aag aag ccc ttt gag gag cac tgg agg aag        1728
Pro Ile Gln Gly Gln Gln Lys Lys Pro Phe Glu Glu His Trp Arg Lys
            565                     570                 575
cac acc ctg tcc tat gtg gac gtt ggc act ggg aag gtc act ctg gaa        1776
His Thr Leu Ser Tyr Val Asp Val Gly Thr Gly Lys Val Thr Leu Glu
            580                     585                 590
tat aga ccc gtg atc gac aaa act ttg aac gag gct gac tgt gcc acc        1824
Tyr Arg Pro Val Ile Asp Lys Thr Leu Asn Glu Ala Asp Cys Ala Thr
            595                     600                 605
gtc ccg cca gcc att cgc tcc tac tga                                    1851
Val Pro Pro Ala Ile Arg Ser Tyr
            610                     615

<210>   6
<211>   616
<212>   PRT
<213>   Homo sapiens
<400>   6
Met Ser Gly Val Arg Gly Leu Ser Arg Leu Leu Ser Ala Arg Arg Leu
1               5                   10                  15
Ala Leu Ala Lys Ala Trp Pro Thr Val Leu Gln Thr Gly Thr Arg Gly
            20                  25                  30
Phe His Phe Thr Val Asp Gly Asn Lys Arg Ala Ser Ala Lys Val Ser
            35                  40                  45
Asp Ser Ile Ser Ala Gln Tyr Pro Val Val Asp His Glu Phe Asp Ala
            50                  55                  60
Val Val Val Gly Ala Gly Gly Ala Gly Leu Arg Ala Ala Phe Gly Leu
65                  70                  75                  80
Ser Glu Ala Gly Phe Asn Thr Ala Cys Val Thr Lys Leu Phe Pro Thr
                85                  90                  95
Arg Ser His Thr Val Ala Ala Gln Leu Glu Asn Tyr Gly Met Pro Phe
                100                 105                 110
Ser Arg Thr Glu Asp Gly Lys Ile Tyr Gln Arg Ala Phe Gly Gly Gln
            115                 120                 125
Ser Leu Lys Phe Gly Lys Gly Gly Gln Ala His Arg Cys Cys Cys Val
            130                 135                 140
Ala Asp Arg Thr Gly His Ser Leu Leu His Thr Leu Tyr Gly Arg Ser
145                 150                 155                 160
Leu Arg Tyr Asp Thr Ser Tyr Phe Val Glu Tyr Phe Ala Leu Asp Leu
                165                 170                 175
Leu Met Glu Asn Gly Glu Cys Arg Gly Val Ile Ala Leu Cys Ile Glu
                180                 185                 190
Asp Gly Ser Ile His Arg Ile Arg Ala Lys Asn Thr Val Val Ala Thr
                195                 200                 205
Gly Gly Tyr Gly Arg Thr Tyr Phe Ser Cys Thr Ser Ala His Thr Ser
            210                 215                 220
Thr Gly Asp Gly Thr Ala Met Ile Thr Arg Ala Gly Leu Pro Cys Gln
225                 230                 235                 240
Asp Leu Glu Phe Val Gln Phe His Pro Thr Gly Ile Tyr Gly Ala Gly
```

42

```
                    245                    250                    255
    Cys Leu Ile Thr Glu Gly Cys Arg Gly Glu Gly Gly Ile Leu Ile Asn
                260                    265                    270
    Ser Gln Gly Glu Arg Phe Met Glu Arg Tyr Ala Pro Val Ala Lys Asp
            275                    280                    285
    Leu Ala Ser Arg Asp Val Val Ser Arg Ser Met Thr Leu Glu Ile Arg
        290                    295                    300
    Glu Gly Arg Gly Cys Gly Pro Glu Lys Asp His Val Tyr Leu Gln Leu
    305                    310                    315                    320
    His His Leu Pro Pro Glu Gln Leu Ala Thr Arg Leu Pro Gly Ile Ser
                    325                    330                    335
    Glu Thr Ala Met Ile Phe Ala Gly Val Asp Val Thr Lys Glu Pro Ile
                340                    345                    350
    Pro Val Leu Pro Thr Val His Tyr Asn Met Gly Gly Ile Pro Thr Asn
                355                    360                    365
    Tyr Lys Gly Gln Val Leu Arg His Val Asn Gly Gln Asp Gln Ile Val
            370                    375                    380
    Pro Gly Leu Tyr Ala Cys Gly Glu Ala Ala Cys Ala Ser Val His Gly
    385                    390                    395                    400
    Ala Asn Arg Leu Gly Ala Asn Ser Leu Leu Asp Leu Val Val Phe Gly
                    405                    410                    415
    Arg Ala Cys Ala Leu Ser Ile Glu Glu Ser Cys Arg Pro Gly Asp Lys
                420                    425                    430
    Val Pro Pro Ile Lys Pro Asn Ala Gly Glu Glu Ser Val Met Asn Leu
                435                    440                    445
    Asp Lys Leu Arg Phe Ala Asp Gly Ser Ile Arg Thr Ser Glu Leu Arg
            450                    455                    460
    Leu Ser Met Gln Lys Ser Met Gln Asn His Ala Ala Val Phe Arg Val
    465                    470                    475                    480
    Gly Ser Val Leu Gln Glu Gly Cys Gly Lys Ile Ser Lys Leu Tyr Gly
                    485                    490                    495
    Asp Leu Lys His Leu Lys Thr Phe Asp Arg Gly Met Val Trp Asn Thr
                500                    505                    510
    Asp Leu Val Glu Thr Leu Glu Leu Gln Asn Leu Met Leu Cys Ala Leu
            515                    520                    525
    Gln Thr Ile Tyr Gly Ala Glu Ala Arg Lys Glu Ser Arg Gly Ala His
            530                    535                    540
    Ala Arg Glu Asp Tyr Lys Val Arg Ile Asp Glu Tyr Asp Tyr Ser Lys
    545                    550                    555                    560
    Pro Ile Gln Gly Gln Gln Lys Lys Pro Phe Glu Glu His Trp Arg Lys
                565                    570                    575
    His Thr Leu Ser Tyr Val Asp Val Gly Thr Gly Lys Val Thr Leu Glu
                580                    585                    590
    Tyr Arg Pro Val Ile Asp Lys Thr Leu Asn Glu Ala Asp Cys Ala Thr
            595                    600                    605
    Val Pro Pro Ala Ile Arg Ser Tyr
        610                    615
<210>   7
<211>   20
<212>   DNA
<213>   Homo sapiens
<400>   7
gctgagcaca ttgagtcacg                                                    20
<210>   8
<211>   20
<212>   DNA
<213>   Homo sapiens
<400>   8
tggtacacct tggatgttgg                                                    20
<210>   9
<211>   24
<212>   DNA
<213>   Homo sapiens
```

<400> 9
tagaaatcgc tgcgtacaga aaac                                              24

<210> 10
<211> 21
<212> DNA
<213> Homo sapiens

<400> 10
tgcttcctgc aaatgtgcta a                                                 21

<210> 11
<211> 20
<212> DNA
<213> Homo sapiens

<400> 11
tgggaacaag agggcatctg                                                   20

<210> 12
<211> 22
<212> DNA
<213> Homo sapiens

<400> 12
ccaccactgc atcaaattca tg                                                22

<210> 13
<211> 834
<212> DNA
<213> Homo sapiens

<220>
<221> CDS
<222> (1)..(834)

<400> 13

```
atg atc atc tta att tac tta ttt ctc ttg cta tgg gaa gac act caa        48
Met Ile Ile Leu Ile Tyr Leu Phe Leu Leu Trp Glu Asp Thr Gln
1               5                   10                  15

gga tgg gga ttc aag gat gga att ttt cat aac tcc ata tgg ctt gaa        96
Gly Trp Gly Phe Lys Asp Gly Ile Phe His Asn Ser Ile Trp Leu Glu
                20                  25                  30

cga gca gcc ggt gtg tac cac aga gaa gca cgg tct ggc aaa tac aag       144
Arg Ala Ala Gly Val Tyr His Arg Glu Ala Arg Ser Gly Lys Tyr Lys
            35                  40                  45

ctc acc tac gca gaa gct aag gcg gtg tgt gaa ttt gaa ggc ggc cat       192
Leu Thr Tyr Ala Glu Ala Lys Ala Val Cys Glu Phe Glu Gly Gly His
        50                  55                  60

ctc gca act tac aag cag cta gag gca gcc aga aaa att gga ttt cat       240
Leu Ala Thr Tyr Lys Gln Leu Glu Ala Ala Arg Lys Ile Gly Phe His
65                  70                  75                  80

gtc tgt gct gct gga tgg atg gct aag ggc aga gtt gga tac ccc att       288
Val Cys Ala Ala Gly Trp Met Ala Lys Gly Arg Val Gly Tyr Pro Ile
                85                  90                  95

gtg aag cca ggg ccc aac tgt gga ttt gga aaa act ggc att att gat       336
Val Lys Pro Gly Pro Asn Cys Gly Phe Gly Lys Thr Gly Ile Ile Asp
                100                 105                 110

tat gga atc cgt ctc aat agg agt gaa aga tgg gat gcc tat tgc tac       384
Tyr Gly Ile Arg Leu Asn Arg Ser Glu Arg Trp Asp Ala Tyr Cys Tyr
            115                 120                 125

aac cca cac gca aag gag tgt ggt ggc gtc ttt aca gat cca aag caa       432
Asn Pro His Ala Lys Glu Cys Gly Gly Val Phe Thr Asp Pro Lys Gln
        130                 135                 140

att ttt aaa tct cca ggc ttc cca aat gag tac gaa gat aac caa atc       480
Ile Phe Lys Ser Pro Gly Phe Pro Asn Glu Tyr Glu Asp Asn Gln Ile
145                 150                 155                 160

tgc tac tgg cac att aga ctc aag tat ggt cag cgt att cac ctg agt       528
Cys Tyr Trp His Ile Arg Leu Lys Tyr Gly Gln Arg Ile His Leu Ser
                165                 170                 175

ttt tta gat ttt gac ctt gaa gat gac cca ggt tgc ttg gct gat tat       576
Phe Leu Asp Phe Asp Leu Glu Asp Asp Pro Gly Cys Leu Ala Asp Tyr
```

```
                     180                    185                    190
        gtt gaa ata tat gac agt tac gat gat gtc cat ggc ttt gtg gga aga       624
        Val Glu Ile Tyr Asp Ser Tyr Asp Asp Val His Gly Phe Val Gly Arg
                     195                    200                    205
        tac tgt gga gat gag ctt cca gat gac atc atc agt aca gga aat gtc       672
        Tyr Cys Gly Asp Glu Leu Pro Asp Asp Ile Ile Ser Thr Gly Asn Val
                     210                    215                    220
        atg acc ttg aag ttt cta agt gat gct tca gtg aca gct gga ggt ttc       720
        Met Thr Leu Lys Phe Leu Ser Asp Ala Ser Val Thr Ala Gly Gly Phe
        225                    230                    235                    240
        caa atc aaa tat gtt gca atg gat cct gta tcc aaa tcc agt caa gga       768
        Gln Ile Lys Tyr Val Ala Met Asp Pro Val Ser Lys Ser Ser Gln Gly
                     245                    250                    255
        aaa aat aca agt act act tct act gga aat aaa aac ttt tta gct gga       816
        Lys Asn Thr Ser Thr Thr Ser Thr Gly Asn Lys Asn Phe Leu Ala Gly
                     260                    265                    270
        aga ttt agc cac tta taa                                               834
        Arg Phe Ser His Leu
                     275

<210>   14
<211>   277
<212>   PRT
<213>   Homo sapiens
<400>   14
Met Ile Ile Leu Ile Tyr Leu Phe Leu Leu Leu Trp Glu Asp Thr Gln
1               5                   10                  15
Gly Trp Gly Phe Lys Asp Gly Ile Phe His Asn Ser Ile Trp Leu Glu
                20                  25                  30
Arg Ala Ala Gly Val Tyr His Arg Glu Ala Arg Ser Gly Lys Tyr Lys
                35                  40                  45
Leu Thr Tyr Ala Glu Ala Lys Ala Val Cys Glu Phe Glu Gly Gly His
        50                  55                  60
Leu Ala Thr Tyr Lys Gln Leu Glu Ala Ala Arg Lys Ile Gly Phe His
65                  70                  75                  80
Val Cys Ala Ala Gly Trp Met Ala Lys Gly Arg Val Gly Tyr Pro Ile
                85                  90                  95
Val Lys Pro Gly Pro Asn Cys Gly Phe Gly Lys Thr Gly Ile Ile Asp
            100                 105                 110
Tyr Gly Ile Arg Leu Asn Arg Ser Glu Arg Trp Asp Ala Tyr Cys Tyr
            115                 120                 125
Asn Pro His Ala Lys Glu Cys Gly Gly Val Phe Thr Asp Pro Lys Gln
        130                 135                 140
Ile Phe Lys Ser Pro Gly Phe Pro Asn Glu Tyr Glu Asp Asn Gln Ile
145                 150                 155                 160
Cys Tyr Trp His Ile Arg Leu Lys Tyr Gly Gln Arg Ile His Leu Ser
                165                 170                 175
Phe Leu Asp Phe Asp Leu Glu Asp Asp Pro Gly Cys Leu Ala Asp Tyr
            180                 185                 190
Val Glu Ile Tyr Asp Ser Tyr Asp Asp Val His Gly Phe Val Gly Arg
            195                 200                 205
Tyr Cys Gly Asp Glu Leu Pro Asp Asp Ile Ile Ser Thr Gly Asn Val
            210                 215                 220
Met Thr Leu Lys Phe Leu Ser Asp Ala Ser Val Thr Ala Gly Gly Phe
225                 230                 235                 240
Gln Ile Lys Tyr Val Ala Met Asp Pro Val Ser Lys Ser Ser Gln Gly
            245                 250                 255
Lys Asn Thr Ser Thr Thr Ser Thr Gly Asn Lys Asn Phe Leu Ala Gly
            260                 265                 270
Arg Phe Ser His Leu
            275
```

**Description**

Technical Field

The present invention relates to a production method of a cell population comprising adherent stem cells such as mesenchymal stem cells. The present invention further relates to a cell population comprising adherent stem cells and a pharmaceutical composition. The present invention further relates to a method for monitoring immunosuppressive activity and/or proliferative property of adherent stem cells using indices for a cell population comprising adherent stem cells, a method for evaluating a donor and/or a biological sample collected from the donor, and a method for determining and/or predicting optimal enzymatic treatment conditions.

Background Art

Adherent stem cells such as mesenchymal stem cells, also called mesenchymal stromal cells, are somatic stem cells reported to exist in the bone marrow, adipose tissues, tooth pulp and the like. Recently, it has been revealed that these cells also exist in fetal appendages including the placenta, umbilical cord, and fetal membrane.
Since the adherent stem cells have immunosuppressive capacity, it has been reported that intravenous administration of a cell preparation comprising mesenchymal stem cells enables treatment of acute graft-versus-host disease (GVHD), Crohn's disease as an inflammatory bowel disease, and the like.
Patent Literature 1 describes a method for producing an amniotic mesenchymal cell composition, a method for cryopreserving the composition, and a therapeutic agent. Particularly, this literature discloses that cryopreserved amniotic mesenchymal cells can be produced as a cell preparation optimized for transplantation, by cryopreserving a mixture comprising amniotic mesenchymal cells in a solution comprising 5 to 10% by mass of dimethyl sulfoxide and comprising 5 to 10% by mass of hydroxyethyl starch or 1 to 5% by mass of dextran.
Patent Literature 2 describes a method for producing a cell population comprising mesenchymal stem cells, comprising a selection step of selecting mesenchymal stem cells having relatively high proliferative capacity by treating a cell population comprising mesenchymal stem cells having different proliferative capacities with a physical stimulus or a chemical stimulus, wherein the selected mesenchymal stem cells having relatively high proliferative capacity is negative for CD106, and the expression level of a metallothionein family gene is high compared to that before the treatment with a physical stimulus or a chemical stimulus.
Patent Literature 3 describes a method for preparing an amniotic mesenchymal stem cell population, comprising the steps of: (D) collecting a cell population of mesenchymal cells from the amnion of a mammal; (E) seeding the collected cell population at a cell concentration of 400 to 35000 cells/cm$^2$, followed by initial culture for 2 to 3 days; (F) seeding the cultured cells at 1/5000 or more and less than 1/10 of the cell concentration of the initial culture, and repeating subculture three to four times with medium replacement twice a week; and (G) maintaining the culture of the cells in the same culture dish until confluence when a colony of cells having a fusiform shape is formed in the subculture.

Citation List

Patent Literatures

Patent Literature 1: JP Patent Publication (Kokai) No. 2015-61520 A
Patent Literature 2: International Publication No. WO 2017/073656
Patent Literature 3: International Publication No. WO 2013/077428

Summary of Invention

Object to be Achieved

In recent years, with an increase of medical expense, a movement of emphasizing economic efficiency of expensive pharmaceutical products represented by biopharmaceuticals has been accelerated. For realizing and spreading cell preparations comprising living cells as an active ingredient, it is important to reduce manufacturing cost of the cell preparations.
In such circumstances, it has been recently found that an adherent stem cell population is a heterogeneous cell population comprising various cells different in differentiation capacity, proliferative capacity and/or cytokine producing capacity. For reducing manufacturing cost of cell preparations, it is necessary to efficiently prepare highly functional adherent stem cells, specifically, an adherent stem cell population exhibiting high therapeutic effect and high proliferative property.

Patent Literature 1 discloses that cryopreserved amniotic mesenchymal cells can be produced as a cell preparation optimized for transplantation, by cryopreserving a mixture comprising amniotic mesenchymal cells in a specific cryopreservation solution to prevent a decrease in the survival rate of amniotic mesenchymal cells after thawing. Patent Literature 2 discloses that mesenchymal stem cells having relatively high proliferative capacity can be prepared by treatment with a physical stimulus or a chemical stimulus. However, Patent Literatures 1 and 2 neither describe nor suggest that mesenchymal stem cells having a specific excellent feature are selectively prepared from mesenchymal stem cells, specifically, that a cell population rich in mesenchymal stem cells exhibiting high immunosuppressive activity and proliferative property is selectively prepared by use of characteristics of the mesenchymal stem cells as indices. Even if manufacturing methods described in Patent Literatures 1 and 2 are used, since the obtained cells do not have sufficient immunosuppressive activity and specific growth rate. Thus, it takes time for cell culture and material cost additionally required. Besides this, since medicinal effect cannot be enhanced without adding a large amount of cells serving as an active ingredient, it is difficult to produce cell preparations at low cost.

In Patent Literature 3, a mesenchymal stem cell population having high proliferative capacity and differentiation capacity is prepared by seeding cells at a low density. However, the literature neither describes nor suggests that a cell population rich in mesenchymal stem cells exhibiting high immunosuppressive activity and proliferative property is selected by using characteristics of the mesenchymal stem cells in a mesenchymal stem cell population as indices. In addition, the mesenchymal stem cell population described in Patent Literature 3 is insufficient in immunosuppressive activity and specific growth rate. Actually, when the present inventors conducted replication studies on the preparation method described in Patent Literature 3. As a result, it was found not only that a step of seeding cells at low density and repeating subculture three to four times requires 23 days but also that the specific growth rate of the mesenchymal stem cell population obtained is as insufficient as 0.34 (1/day). Thus, even if the preparation method described in Patent Literature 3 is used, it is impossible to reduce the period of cell culture and difficult to produce a cell preparation at low cost.

An object of the present invention is to provide a cell population comprising adherent stem cells exhibiting high immunosuppressive activity and/or proliferative property useful for reducing manufacturing cost of a cell preparation, and a production method thereof, and provide a pharmaceutical composition comprising the cell population. Another object of the present invention is to provide a method for monitoring immunosuppressive activity and/or proliferative property of adherent stem cells by using indices on a cell population comprising adherent stem cells, a method for evaluating a donor and/or a biological sample collected from the donor, and a method for determining and/or predicting optimal enzymatic treatment conditions.

Means to Achieve the Object

The present inventors conducted intensive studies with a view to achieving the above object. As a result, it was found that adherent stem cells, in which expressions of PLIN2 gene and NEFM gene are positive and the relative expression level of PLIN2 gene to the expression level of SDHA gene is 1.50 or more, are included in a cell population comprising adherent stem cells; further it was found that the cell population comprising adherent stem cells having the above cell characteristics exhibits high immunosuppressive activity. The present inventors further found that the cell population comprising adherent stem cells having the above cell characteristics has a high specific growth rate. The present inventors further found that, in a cell population comprising adherent stem cells, the immunosuppressive activity and/or proliferative property of the adherent stem cells can be monitored by using positive expressions of PLIN2 gene and NEFM gene, and the relative expression level of PLIN2 gene to the expression level of SDHA gene of 1.50 or more, as indices. Furthermore, from the viewpoint of efficiently obtaining adherent stem cells exhibiting high immunosuppressive activity and/or proliferative property, it was found that the quality of a donor and/or a biological sample collected from the donor can be evaluated. Moreover, from the viewpoint of efficiently obtaining adherent stem cells exhibiting high immunosuppressive activity and/or proliferative property, it was found that optimal enzymatic treatment conditions for a biological sample collected from a donor can be determined and/or predicted. The present invention was accomplished on the basis of these findings.

Accordingly, the following inventions are provided herein.

[1] A production method a cell population comprising adherent stem cells, comprising obtaining a cell population having the following cell characteristics (a) and (b):

(a) the cell population is positive for expressions of PLIN2 gene and NEFM gene,
(b) the cell population satisfies the relative expression level of PLIN2 gene to the expression level of SDHA gene of 1.50 or more.

[2] The production method according to [1], wherein the cell population satisfies the relative expression level of NEFM gene to the expression level of SDHA gene of less than 0.02.

[3] The production method according to [1] or [2], wherein the cell population satisfies the relative expression level of PLIN2 gene to the expression level of NEFM gene of 250 or more.

[4] A production method of a cell population comprising mesenchymal stem cells, comprising obtaining a cell population having the following cell characteristics (a) and (b):

(a) the cell population satisfies the relative expression level of PLIN2 gene to the expression level of SDHA gene of more than 0.30,
(b) the cell population satisfies the relative expression level of NEFM gene to the expression level of SDHA gene of less than 1.50.

[5] A cell population comprising adherent stem cells and having the following cell characteristics (a) and (b):

(a) the cell population is positive for expressions of PLIN2 gene and NEFM gene,
(b) the cell population satisfies the relative expression level of PLIN2 gene to the expression level of SDHA gene of 1.50 or more.

[6] The cell population according to [5], wherein the cell population satisfies the relative expression level of NEFM gene to the expression level of SDHA gene of less than 0.02.

[7] The cell population according to [5] or [6], wherein the cell population satisfies the relative expression level of PLIN2 gene to the expression level of NEFM gene of 250 or more.

[8] A cell population comprising mesenchymal stem cells and having the following cell characteristics (a) and (b):

(a) the cell population satisfies the relative expression level of PLIN2 gene to the expression level of SDHA gene of more than 0.30,
(b) the cell population satisfies the relative expression level of NEFM gene to the expression level of SDHA gene of less than 1.50.

[9] The cell population according to any one of [5] to [8], wherein the adherent stem cells are derived from a fetal appendage.

[10] A pharmaceutical composition comprising the cell population according to any one of [5] to [9] and a pharmaceutically acceptable vehicle.

[11] The pharmaceutical composition comprising the cell population according to any one of [5] to [9] and additional cells that can be administered.

[12] The pharmaceutical composition according to [10] or [11], wherein a single dose of the adherent stem cells to a human is $1 \times 10^{12}$ cells/kg body weight or less.

[13] The pharmaceutical composition according to any one of [10] to [12], wherein the pharmaceutical composition is an injectable preparation.

[14] The pharmaceutical composition according to any one of [10] to [12], wherein the pharmaceutical composition is a transplant preparation of a cell aggregate or sheet-like structure.

[15] The pharmaceutical composition according to any one of [10] to [14], being a therapeutic agent for an immune-related disease.

[16] The cell population according to any one of [5] to [9], wherein the specific growth rate is 0.40 (1/day) or more.

[17] The cell population according to any one of [5] to [9], wherein the cell population satisfies the relative expression level of TNFAIP6 gene to the expression level of SDHA gene of 0.5 or more.

[18] A cell population obtained by the production method according to any one of [1] to [4].

[18-2] The production method according to any one of [1] to [4], wherein the cell population satisfies the relative expression level of TNFAIP6 gene to the expression level of SDHA gene of 0.5 or more.

[19] Use of the cell population according to any one of [5] to [9] for the manufacture of a pharmaceutical composition.

[20] Use according to [19], wherein the pharmaceutical composition is a pharmaceutical composition where a single dose of the adherent stem cells to a human is $1 \times 10^{12}$ cells/kg body weight or less.

[21] Use according to [19] or [20], wherein the pharmaceutical composition is an injectable preparation.

[22] Use according to [19] or [20], wherein the pharmaceutical composition is a transplant preparation of a cell aggregate or sheet-like structure.

[23] Use according to any one of [19] to [22], wherein the pharmaceutical composition is a therapeutic agent for an immune-related disease.

[24] The cell population according to any one of [5] to [9], for use in the treatment of a disease.

[25] The cell population according to [24], wherein a single dose of the adherent stem cells to a human is $1 \times 10^{12}$ cells/kg body weight or less.

[26] The cell population according to [24] or [25], being an injectable preparation.

[27] The cell population according to [24] or [25], being a transplant preparation of a cell aggregate or sheet-like structure.

[28] The cell population according to any one of [24] to [27], wherein the disease is an immune-related disease.

[29] A method for treating a disease, comprising administering the cell population according to any one of [5] to [9] to a patient or subject in need of treatment.

[30] The method for treating a disease according to [29], wherein a single dose of the adherent stem cells to a human is $1 \times 10^{12}$ cells/kg body weight or less.

[31] The method for treating a disease according to [29] or [30], being an injectable preparation.

[32] The method for treating a disease according to [29] or [30], being a transplant preparation of a cell aggregate or sheet-like structure.

[33] The method for treating a disease according to any one of [29] to [32], wherein the disease is an immune-related disease.

[34] A composition comprising the cell population according to any one of [5] to [9] and a vehicle.

[35] A method for monitoring immunosuppressive activity and/or proliferative property of adherent stem cells in a cell population comprising adherent stem cells, comprising: determining the presence or absence of expressions of PLIN2 gene and NEFM gene and measuring the relative expression level of PLIN2 gene to the expression level of SDHA gene; and using the following cell characteristics (a) and (b) as indices:

(a) the cell population is positive for expressions of PLIN2 gene and NEFM gene,
(b) the cell population satisfies the relative expression level of PLIN2 gene to the expression level of SDHA gene of 1.50 or more.

[36] A method for monitoring proliferative property and/or immunosuppressive activity of mesenchymal stem cells in a cell population comprising mesenchymal stem cells, using the following cell characteristics (a) and (b) as indices, the method comprising measuring the relative expression level of PLIN2 gene to the expression level of SDHA gene and the relative expression level of NEFM gene to the expression level of SDHA gene:

(a) the cell population satisfies the relative expression level of PLIN2 gene to the expression level of SDHA gene of more than 0.30,
(b) the cell population satisfies the relative expression level of NEFM gene to the expression level of SDHA gene of less than 1.50.

[37] A method for evaluating a donor and/or a biological sample collected from the donor, comprising: collecting a cell population comprising adherent stem cells from the donor; determining the presence or absence of expressions of PLIN2 gene and NEFM gene and measuring the relative expression level of PLIN2 gene to the expression level of SDHA gene; and evaluating the relative expression level by using the following cell characteristics (a) and (b) as indices:

(a) the cell population is positive for expressions of PLIN2 gene and NEFM gene,
(b) the cell population satisfies the relative expression level of PLIN2 gene to the expression level of SDHA gene of 1.50 or more.

[38] A method for evaluating a donor and/or a biological sample collected from the donor, comprising: collecting a cell population comprising mesenchymal stem cells from the donor; measuring the relative expression level of PLIN2 gene to the expression level of SDHA gene and the relative expression level of NEFM gene to the expression level of SDHA gene; and evaluating the relative expression levels by using the following cell characteristics (a) and (b) as indices:

(a) the cell population satisfies the relative expression level of PLIN2 gene to the expression level of SDHA gene of more than 0.30,
(b) the cell population satisfies the relative expression level of NEFM gene to the expression level of SDHA gene of less than 1.50.

[39] A method for determining and/or predicting an optimal condition for enzymatic treatment for a biological sample collected from a donor, the method comprising determining the presence or absence of expressions of PLIN2 gene and NEFM gene; measuring the relative expression level of PLIN2 gene to the expression level of SDHA gene for the cell population obtained by enzymatically treating the sample; and evaluating the relative expression level by using the following cell characteristics (a) and (b) as indices:

(a) the cell population is positive for expressions of PLIN2 gene and NEFM gene,
(b) the cell population satisfies the relative expression level of PLIN2 gene to the expression level of SDHA gene of 1.50 or more.

[40] A method for determining and/or predicting an optimal condition for enzymatic treatment for a biological sample collected from a donor, the method comprising measuring the relative expression level of PLIN2 gene to the expression level of SDHA gene and the relative expression level of NEFM gene to the expression level of SDHA gene for the cell population obtained by enzymatically treating the sample; and evaluating the relative expression levels by using the following cell characteristics (a) and (b) as indices:

(a) the cell population satisfies the relative expression level of PLIN2 gene to the expression level of SDHA gene of more than 0.30,
(b) the cell population satisfies the relative expression level of NEFM gene to the expression level of SDHA gene of less than 1.50.

Embodiments for Carrying out the Invention

According to the present invention, a cell population comprising adherent stem cells exhibiting high immunosuppressive activity can be obtained. In addition, according to the present invention, a cell population comprising adherent stem cells exhibiting high proliferative property can be obtained. In addition, according to the present invention, a means of monitoring high immunosuppressive activity and/or proliferative property in a cell population comprising adherent stem cells can be provided. In addition, according to the present invention, relative expression levels of various genes to a housekeeping gene can be used as indices for formation of a cell population comprising adherent stem cells exhibiting high immunosuppressive activity and/or proliferative property. This enables a manufacture of a cell preparation (pharmaceutical composition) at low cost.

Brief Description of Drawings

[Figure 1] Figure 1 shows results of viability of Example 2.
[Figure 2] Figure 2 shows results of viability of Example 7.

Description of Embodiments

Embodiments of the present invention will be specifically described below. The descriptions below are intended to facilitate understanding of the present invention. Thus, the scope of the present invention is not limited to the following embodiments. Other embodiments, which a person skilled in the art may obtain as appropriate by replacing features of the following embodiments, are also included in the scope of the present invention.

[1] Explanation of terms

The term "fetal appendage" used herein refers to a fetal membrane, a placenta, an umbilical cord, and amniotic fluid. In addition, the term "fetal membrane" refers to a fetal sac comprising fetal amniotic fluid, which consists of an amnion, a chorion, and a decidua in that order from the inside. Among them, the amnion and the chorion are originated from the fetus. The term "amnion" refers to a transparent thin membrane with few blood vessels, which is located in the most inner layer of the fetal membrane. The inner layer (also called epithelial cell layer) of the amnion is covered with a layer of epithelial cells having a secretory function and secretes amniotic fluid. The outer layer (also called extracellular matrix layer, which corresponds to the stroma) of the amnion comprises adherent stem cells.
The term "adherent stem cells" used herein refers to stem cells that satisfy the definition described below and "mesenchymal stromal cells" and "mesenchymal stem cells" are also included in the adherent stem cells. The term "mesenchymal stem cells" is also referred to as "MSCs" in the specification.
As the term "adherent stem cells", somatic stem cells (tissue stem cells) taken from various tissues and organs and satisfying the following definition can be used. Examples of the somatic stem cells (tissue stem cells) include, but are not particularly limited to, bone marrow-derived mesenchymal stem cells, hematopoietic stem cells, stem cells in umbilical blood, umbilical cord-derived stem cells, amnion-derived stem cells, amniotic fluid stem cells, placental villus cell-derived mesenchymal stem cells, neural stem cells, adipose tissue-derived stem cells, pancreatic stem cells, synovial mesenchymal stem cells, dental pulp stem cells, stem cells from pulp from deciduous milk teeth, germline stem cells (GS cells), multipotent germline stem cells (mGS cells), corneal epithelial stem cells, corneal parenchymal stem cells, pigment stem cells and tissue stem cells in organs.

Definition of adherent stem cells

i) Adherence to plastics under culture conditions in a standard medium.
ii) Positive for surface antigens CD73 and CD90, and negative for surface antigen CD326.

The term "adherent stem cell population" used herein means a cell population comprising adherent stem cells. Examples of the form thereof include, but are not particularly limited to, cell pellets, cell sheets, cell aggregates, cell-floated liquids and cell suspensions.

The "adherent stem cells" are not particularly limited as long as they satisfy the above definitions and are not particularly restricted by the presence or absence of differentiation capacity into bone, cartilage, adipose and the like. For example, cells having differentiation capacity into bone, cartilage and adipose, like mesenchymal stem cells are included in the "adherent stem cells" herein. The cells that satisfy the above definitions but fail to have differentiation capacity into bone, cartilage, adipose and the like are also included in the "adherent stem cells". The cells that satisfy the above definitions and differentiate only into any one or two of bone, cartilage and adipose are also included in the "adherent stem cells".

The term "amniotic adherent stem cells" herein refers to adherent stem cells derived from the amnion and are used interchangeably with "amniotic mesenchymal stromal cells". The term "amniotic mesenchymal stem cells" used herein is also referred to as "amniotic MSCs".

The phrase "proportion of surface antigen-positive adherent stem cells" used herein refers to the proportion of cells positive for the surface antigen analyzed by flow cytometry, as described in Examples mentioned later. The phrase "proportion of surface antigen-positive adherent stem cells" is also referred to as a "positive rate" in the specification.

The phrase "proportion of surface antigen-negative adherent stem cells" used herein refers to the proportion of cells negative for the surface antigen analyzed by flow cytometry as described in Examples mentioned later. The phrase "proportion of surface antigen-negative adherent stem cells" is also referred to as "negative rate" in the specification.

The term "proliferative capacity" used herein refers to a capacity of cells to proliferate by cell division. The phrase "proliferative capacity is high" is used herein interchangeably with "proliferative property is high". The proliferative capacity of adherent stem cell population can be evaluated by using the obtained cell number per batch of culture, specific growth rate, number of doublings, doubling time, and/or the number of passages. The measuring method for specific growth rate is as described later in the specification.

The "obtained cell number per batch of culture" used herein means the number of cells obtained per unit surface area of a culture vessel per unit number of culture days in a single culture. Accordingly, the unit of the "obtained cell number per batch of culture" is (cells/cm$^2$/day).

The term "immunosuppressive activity" used herein refers to an effect to suppress immune response of a living body. The term "immunosuppressive activity" can be used herein interchangeably with "immunosuppressive capacity". The immunosuppressive activity of the adherent stem cell population can be evaluated by expression level of TNFAIP6 (tumor necrosis factor, alpha-induced protein 6) gene, proliferation inhibition rate of human peripheral blood mononuclear cells (hPBMCs) in a mixed lymphocyte reaction test (MLR test) and animal experimentation. The evaluation method of immunosuppressive activity is as described later in the specification.

[2] Cell population comprising adherent stem cells

The cell population comprising adherent stem cells and provided by the present invention is characterized by

(a) the cell population is positive for expressions of PLIN2 gene and NEFM gene,
(b) the cell population satisfies the relative expression level of PLIN2 gene to the expression level of SDHA gene of 1.50 or more.

If the cell population comprising adherent stem cells and provided by the present invention satisfies the conditions of (a) the cell population is positive for expressions of PLIN2 gene and NEFM gene and (b) the cell population satisfies the relative expression level of PLIN2 gene to the expression level of SDHA gene of 1.50 or more, a cell population comprising adherent stem cells exhibiting high immunosuppressive activity and/or proliferative property can be formed. Thus, in the present invention, the above conditions can be used as indices for the formation of a cell population exhibiting high immunosuppressive activity and/or proliferative property. Also, if the indices are measured over time, changes in the immunosuppressive activity and/or proliferative property of adherent stem cells can be quickly determined and predicted. According to the present invention, the quality of a donor itself and/or a biological sample collected from the donor can be evaluated by using the indices. Further, according to the present invention, whether or not an enzymatic treatment method is appropriate for enzymatically treating a biological sample collected from a donor can be determined and/or predicted by using the indices.

PLIN2 stands for Perilipin 2. The sequence of PLIN2 gene is registered as ID: 123 in the gene database of the National Center for Biotechnology Information. PLIN2 is a gene consisting of the nucleotide sequence represented by SEQ ID NO: 1 or a gene encoding a polypeptide consisting of the amino acid sequence represented by SEQ ID NO: 2.

NEFM stands for a neurofilament medium polypeptide. The sequence of NEFM gene is registered as ID: 4741 in the gene database of the National Center for Biotechnology Information. NEFM is a gene consisting of the nucleotide sequence represented by SEQ ID NO: 3 or a gene encoding a polypeptide consisting of the amino acid sequence represented by SEQ ID NO: 4.

SDHA is a kind of housekeeping gene and stands for succinate dehydrogenase complex, subunit A. The sequence of SDHA gene is registered as ID: 6389 in the gene database of the National Center for Biotechnology Information. SDHA is a gene consisting of the nucleotide sequence represented by SEQ ID NO: 5 or a gene encoding a polypeptide consisting of the amino acid sequence represented by SEQ ID NO: 6.

The upper limit of the relative expression level of PLIN2 gene to the expression level of SDHA gene may be, for example, 20.0 or less, 19.0 or less, 18.0 or less, 17.0 or less, 16.0 or less, 15.0 or less, 14.0 or less, 13.0 or less, 12.0 or less, 11.0 or less, 10.0 or less, 9.0 or less, 8.9 or less, 8.8 or less, 8.7 or less, 8.6 or less, 8.5 or less, 8.4 or less, 8.3 or less, 8.2 or less, 8.1 or less, 8.0 or less, 7.99 or less, 7.98 or less, 7.97 or less, 7.96 or less, 7.95 or less, 7.94 or less, 7.8 or less, 7.7 or less, 7.6 or less, 7.5 or less, 7.4 or less, 7.3 or less, 7.2 or less, 7.1 or less, 7.0 or less, 6.9 or less, 6.8 or less, 6.7 or less, 6.6 or less, 6.5 or less, 6.4 or less, 6.3 or less, 6.2 or less, 6.1 or less, 6.0 or less, 5.9 or less, 5.8 or less, 5.7 or less, 5.6 or less, 5.5 or less, 5.4 or less, 5.3 or less, 5.2 or less, 5.1 or less, or 5.0 or less.

The lower limit of the relative expression level of PLIN2 gene to the expression level of SDHA gene may be, for example, 1.51 or more, 1.52 or more, 1.53 or more, 1.54 or more, 1.55 or more, 1.56 or more, 1.57 or more, 1.58 or more, 1.59 or more, 1.60 or more, 1.61 or more, 1.62 or more, 1.63 or more, 1.64 or more, 1.65 or more, 1.66 or more, 1.67 or more, 1.68 or more, 1.69 or more, 1.70 or more, 1.71 or more, 1.72 or more, 1.73 or more, 1.74 or more, 1.75 or more, 1.76 or more, 1.77 or more, 1.78 or more, 1.79 or more, 1.80 or more, 1.81 or more, 1.82 or more, 1.83 or more, 1.84 or more, 1.85 or more, 1.86 or more, 1.87 or more, 1.88 or more, 1.89 or more, 1.90 or more, 1.91 or more, 1.92 or more, 1.93 or more, 1.94 or more, 2.0 or more, 2.1 or more, 2.2 or more, 2.3 or more, 2.4 or more, 2.5 or more, 2.6 or more, 2.7 or more, 2.8 or more, 2.9 or more, 3.0 or more, 3.1 or more, 3.2 or more, 3.3 or more, 3.4 or more, 3.5 or more, 3.6 or more, 3.7 or more, 3.8 or more, 3.9 or more, 4.0 or more, 4.1 or more, 4.2 or more, 4.3 or more, 4.4 or more, 4.5 or more, 4.6 or more, 4.7 or more, 4.8 or more, 4.9 or more, or 5.0 or more.

The upper limit of the relative expression level of NEFM gene to the expression level of SDHA gene may be, for example, less than 0.020, 0.019 or less, 0.018 or less, 0.017 or less, 0.016 or less, 0.015 or less, 0.014 or less, 0.013 or less, 0.012 or less, 0.011 or less, 0.010 or less, 0.009 or less, or 0.008 or less.

The lower limit of the relative expression level of NEFM gene to the expression level of SDHA gene may be, for example, 0.0001 or more, 0.0002 or more, 0.0003 or more, 0.0004 or more, 0.0005 or more, 0.0006 or more, 0.0007 or more, 0.0008 or more, 0.0009 or more, 0.001 or more, 0.002 or more, 0.003 or more, 0.004 or more, 0.005 or more, 0.006 or more, 0.007 or more, or 0.008 or more.

The upper limit of the relative expression level of PLIN2 gene to the expression level of NEFM gene may be, for example, 40000 or less, 45000 or less, 30000 or less, 25000 or less, 20000 or less, 15000 or less, 10000 or less, 9000 or less, 8000 or less, 7000 or less, 6000 or less, 5000 or less, 4900 or less, 4800 or less, 4700 or less, 4600 or less, 4500 or less, 4400 or less, 4300 or less, 4200 or less, 4100 or less, 4000 or less, 3900 or less, 3800 or less, 3700 or less, 3600 or less, 3500 or less, 3400 or less, 3300 or less, 3200 or less, 3100 or less, 3090 or less, 3080 or less, 3070 or less, 3060 or less, 3050 or less, 3040 or less, 3030 or less, 3020 or less, 3010 or less, 3000 or less, 2900 or less, 2800 or less, 2700 or less, 2600 or less, 2500 or less, 2400 or less, 2300 or less, 2200 or less, 2100 or less, 2000 or less, 1900 or less, 1800 or less, 1700 or less, 1600 or less, 1500 or less, 1400 or less, 1300 or less, 1200 or less, 1100 or less, or 1000 or less.

The lower limit of the relative expression level of PLIN2 gene to the expression level of NEFM gene may be, for example, 250 or more, 260 or more, 270 or more, 280 or more, 290 or more, 300 or more, 310 or more, 320 or more, 330 or more, 340 or more, 350 or more, 360 or more, 370 or more, 380 or more, 390 or more, 400 or more, 410 or more, 420 or more, 430 or more, 440 or more, 450 or more, 460 or more, 470 or more, 480 or more, 490 or more, 500 or more, 600 or more, 700 or more, 800 or more, 900 or more, or 1000 or more.

Detection of individual genes and/or measurement of expression levels thereof can be carried out, for example, by a microarray, RT-PCR, quantitative RT-PCR or northern blot hybridization, but are not limited to these. As a method for measuring the relative expression levels of individual genes to the expression level of SDHA gene and the relative expression level of PLIN2 gene to the expression level of NEFM gene, a microarray can be used. The microarray can be performed specifically by the following procedures (1) to (5). The following procedures (3) to (5) can be entrusted to and performed by RIKEN GENESIS Co., Ltd.

(1) A cryopreserved cell population is thawed and centrifugally collected. The collected cell population is washed with phosphate buffer (PBS) and centrifugally collected.
(2) Total RNA is extracted from the cell population and purified by RNA extraction kit (RNeasy Plus Mini kit (manufactured by QIAGEN)).
(3) Using the purified total RNA as a template, cDNA is synthesized by reverse transcription. Then, the synthesized cDNA is further transcribed to cRNA by *in vitro* transcription with biotin labeling.
(4) The biotin-labeled cRNA is added to a hybridization buffer and subjected to hybridization on Human GeneGenome U133A 2.0 Array (manufactured by Affymetrix, Inc.) for 16 hours, followed by washing with GeneChip Fluidics Station 450 (manufactured by Affymetrix, Inc.), staining with phycoerythrin, scanning using GeneChip Scanner 3000 7G (manufactured by Affymetrix, Inc.), image analysis using AGCC (Affymetrix GeneChip Command Console Software, manufactured by Affymetrix, Inc.), and then quantification using Affymetrix Expression Console (manufactured by Affymetrix, Inc.).
(5) Numerical data files are compared and analyzed using the analysis software GeneSpring GX (manufactured by Agilent Technologies, Inc.). The relative expression levels of individual genes to the expression level of SDHA gene or NEFM gene in each cell population are calculated.

As the method for measuring the relative expression levels of individual genes to the expression level of SDHA gene and the relative expression level of PLIN2 gene to the expression level of NEFM gene, quantitative RT-PCR can be used. The quantitative RT-PCR can be carried out specifically in accordance with the following procedure A (comparison Ct method) or procedure B (calibration method).

(Procedure A: comparative Ct method)

(1) A cell population cryopreserved is thawed and centrifugally collected. The collected cell population is washed with phosphate buffer (PBS) and centrifugally collected.
(2) Total RNA is extracted from the cell population and purified by RNA extraction kit (RNeasy Plus Mini kit (manufactured by QIAGEN)).
(3) Using the purified total RNA as a template and ReverTra Ace qPCR RT Master Mix with gDNA Remover (manufactured by Toyobo Co., Ltd.), cDNA is synthesized by reverse transcription.
(4) Using synthesized cDNA as a template and KOD SYBR qPCR Mix (manufactured by Toyobo Co., Ltd.), a PCR is carried out. The volume of a reaction solution is set at 20 $\mu$L. The PCR is carried out by use of StepOnePlus Real-Time PCR System (manufactured by Thermo Fisher Scientific Inc.) in the conditions: 98°C, 2 minutes hold, (98°C 10 seconds, 60°C 10 seconds, 68°C 30 seconds) 40 cycles.
(5) The relative expression level (X) of a measurement target gene to a comparative gene is calculated in accordance with $X = 2^{\{-(Y-Z)\}}$, where Y represents the Ct value of a measurement target gene and Z represents the Ct value of a comparative gene.

(Procedure B: calibration method)

(1) A cryopreserved cell population is thawed and centrifugally collected. The collected cell population is washed with phosphate buffer (PBS) and centrifugally collected.
(2) Total RNA is extracted from the cell population and purified by RNA extraction kit (RNeasy Plus Mini kit (manufactured by QIAGEN)).
(3) Using the purified total RNA as a template and ReverTra Ace qPCR RT Master Mix with gDNA Remover (manufactured by Toyobo Co., Ltd.), cDNA is synthesized by reverse transcription.
(4) Using synthesized cDNA as a template and KOD SYBR qPCR Mix (manufactured by Toyobo Co., Ltd.), a PCR is carried out. The volume of a reaction solution is set at 20 μL. The PCR is carried out by use of StepOnePlus Real-Time PCR System (manufactured by Thermo Fisher Scientific Inc.) in the conditions: 98°C, 2 minutes hold, (98°C 10 seconds, 60°C 10 seconds, 68°C 30 seconds) 40 cycles. Note that, as a sample for preparing a calibration curve, synthesized cDNA is subjected to double-fold serial dilution one to five times, and put in use.

(5) The expression levels of the comparative gene and measurement target gene displayed by StepOnePlus Real-Time PCR System (manufactured by Thermo Fisher Scientific Inc.) are recorded.

(6) The relative expression level (W) of the measurement target gene to the comparative gene is calculated in accordance with W = $\alpha/\beta$, where $\alpha$ represents the expression level of a measurement target gene and $\beta$ represents the expression level of a comparative gene.

In the above quantitative RT-PCR, the following primer sequences can be used.

PLIN2-f: 5-GCTGAGCACATTGAGTCACG-3 (SEQ ID NO: 7)
PLIN2-r: 5-TGGTACACCTTGGATGTTGG-3 (SEQ ID NO: 8)
NEFM-f: 5-TAGAAATCGCTGCGTACAGAAAAC-3 (SEQ ID NO: 9)
NEFM-r: 5-TGCTTCCTGCAAATGTGCTAA-3 (SEQ ID NO: 10)
SDHA-f: 5-TGGGAACAAGAGGGCATCTG-3 (SEQ ID NO: 11)
SDHA-r: 5-CCACCACTGCATCAAATTCATG-3 (SEQ ID NO: 12)

The timing for measuring the above gene expression levels is not particularly limited and examples thereof include immediately after separation of cells from a biological sample, during a culture step, after purification in the culture step, immediately after the nth passage (n represents an integer of 1 or more), during maintenance culture, before cryopreservation or after thawing and before formulation as a pharmaceutical composition.

According to an aspect of the present invention, the cell population comprising adherent stem cells and provided by the present invention may satisfy a proportion of CD105, CD73, and/or CD90-positive adherent stem cells of 90% or more.

CD105, which means cluster of differentiation 105, is a protein also known as Endoglin.

CD73, which means cluster of differentiation 73, is a protein also known as 5-Nucleotidase or Ecto-5'-nucleotidase.

CD90, which means cluster of differentiation 90, is a protein also known as Thy-1.

The proportion of CD105-positive adherent stem cells in a cell population may be 91% or more, 92% or more, 93% or more, 94% or more, 95% or more, 96% or more, 97% or more, 98% or more, 99% or more, or 100%.

The proportion of CD73-positive adherent stem cells in a cell population may be 91% or more, 92% or more, 93% or more, 94% or more, 95% or more, 96% or more, 97% or more, 98% or more, 99% or more, or 100%.

The proportion of CD90-positive adherent stem cells in a cell population may be 91% or more, 92% or more, 93% or more, 94% or more, 95% or more, 96% or more, 97% or more, 98% or more, 99% or more, or 100%.

According to an aspect of the present invention, the cell population comprising adherent stem cells and provided by the present invention may satisfy a proportion of CD166-positive adherent stem cells of 30% or more.

CD 166, which means cluster of differentiation 166, is a protein also known as Activaed leukocyte cell adhesion molecule (ALCAM).

The proportion of CD166-positive adherent stem cells in a cell population may be 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, or 100%.

According to an aspect of the present invention, the cell population comprising adherent stem cells and provided by the present invention may satisfy a proportion of CD45, CD34, CD11b, CD79alpha, CD19, and/or HLA-DR-negative adherent stem cells of 95% or more.

CD45, which means cluster of differentiation 45, is a protein also known as PTPRC (Protein tyrosine phosphatase, receptor type C) or LCA (Leukocyte common antigen).

CD34, which means cluster of differentiation 34, is a protein also known as Hematopoietic progenitor cell antigen CD34.

CD11b, which means cluster of differentiation 11b, is a protein also known as ITGAM (Integrin, alpha M).

CD79alpha, which means cluster of differentiation 79alpha, is a protein also known as B-cell antigen receptor complex-associated protein alpha chain or MB-1 membrane glycoprotein.

CD19, which means cluster of differentiation 19, is a protein also known as B-lymphocyte antigen CD19.

HLA-DR, which means Human leukocyte antigen-antigen D related, is a protein also known as MHC class II cell surface receptor.

The proportion of CD45-negative adherent stem cells in a cell population may be 96% or more, 97% or more, 98% or more, 99% or more, or 100%.

The proportion of CD34-negative adherent stem cells in a cell population may be 96% or more, 97% or more, 98% or more, 99% or more, or 100%.

The proportion of CD11b-negative adherent stem cells in a cell population may be 96% or more, 97% or more, 98% or more, 99% or more, or 100%.

The proportion of CD79alpha-negative adherent stem cells in a cell population may be 96% or more, 97% or more, 98% or more, 99% or more, or 100%.

The proportion of CD19-negative adherent stem cells in a cell population may be 96% or more, 97% or more, 98% or more, 99% or more, or 100%.

The proportion of HLA-DR-negative adherent stem cells in a cell population may be 96% or more, 97% or more, 98% or more, 99% or more, or 100%.

According to an aspect of the present invention, the cell population comprising adherent stem cells and provided by the present invention may satisfy a proportion of CD324 and/or CD326-negative adherent stem cells of 95% or more.

CD324, which means cluster of differentiation 324, is a protein also known as Cadherin 1, or E-cadherin encoded by CDH1 gene.

CD326, which means cluster of differentiation 326, is a protein also known as an epithelial cell adhesion molecule encoded by EPCAM gene.

The proportion of CD324-negative adherent stem cells in a cell population may be 96% or more, 97% or more, 98% or more, 99% or more, or 100%.

The proportion of CD326-negative adherent stem cells in a cell population may be 96% or more, 97% or more, 98% or more, 99% or more, or 100%.

Measurement of individual surface antigens can be made by, for example, flow cytometry or cell staining but is not limited to these. When cells that emit stronger fluorescence as compared with a negative control (isotype control) are detected in flow cytometry using a fluorescently labeled antibody, the cells are determined to be "positive" for the surface antigen. Any antibody can be used as the fluorescently labeled antibody. Examples thereof include, but are not limited to, antibodies labeled with fluorescein isothiocyanate (FITC), phycoerythrin (PE) and allophycocyanin (APC). When cells that are colored or emit fluorescence were observed under a microscope in cell staining, the cells are determined to be "positive" for the surface antigen. The cell staining may be immunostaining of cells using an antibody, or may be non-immunostaining of cells not using an antibody.

The proportion of cells positive for each surface antigen (positive rate) can be determined specifically by using flow cytometry dot-plot analysis by the following procedures (1) to (8):

(1) A cryopreserved cell population is thawed and centrifugally collected. The collected cell population is washed with phosphate buffer (PBS) and centrifugally collected.

(2) The cell population is fixed with 4% paraformaldehyde and then washed with phosphate buffer (PBS), and a cell suspension is prepared in 2% BSA/PBS at $1.0 \times 10^6$ cells/mL. The cell suspension is dispensed in 100 μL each.

(3) The dispensed cell suspensions are centrifuged, and 100 μL of 0.5% BSA/PBS is each added to the obtained cell pellets, followed by addition of antibodies against the respective surface antigens or the isotype control antibodies thereof. Each reaction solution is mixed and then allowed to stand at 4°C for 20 minutes.

(4) The cell population is washed by addition of 0.5% BSA/PBS and centrifugation, suspended in 0.5% BSA/PBS and filtered through a cell strainer (35-μm-nylon mesh filter) (Corning Inc./Product number: 352235).

(5) The cell suspension obtained by filtration is analyzed on a BD Accuri™ C6 Flow Cytometer (manufactured by Becton, Dickinson and Company) with ALL Event 10000.

(6) The measurement results are plotted as dots with SSC (side scattered light) (numerical range: 0 or more and 16777215 or less) on the vertical axis and fluorescence intensity of the dye labeled to the antibody (numerical range: $10^1$ or more and $10^{7.2}$ or less) on the horizontal axis.

(7) In the dot plot diagram, all regions (gates) in which the cell population with stronger fluorescence intensity is 1.0% or less from all cells measured with the isotype control antibodies are selected.

(8) The proportion of cells comprised in the gate selected in (7) among all cells measured with the antibody against each surface antigen is calculated.

Note that, the proportion of cells negative for each surface antigen (negative rate) is calculated by the following equation:

$$\text{Negative rate (\%)} = 100 - \text{positive rate}$$

The timing for detecting the surface antigen described above is not particularly limited, and examples thereof include immediately after separation of cells from a biological sample, during a culture step, after purification in the culture step, immediately after n times passages (n represents an integer of 1 or more), during maintenance culture, before cryopreservation, after thawing, or before formulation as a pharmaceutical composition.

The proliferative capacity of the cell population comprising adherent stem cells and provided by the present invention can be evaluated based on the specific growth rate. The specific growth rate is defined as an increase of cell number per unit time and represented by Specific growth rate: $\mu = \ln(mt2/mt1)/(t2-t1)$

where t1 and t2 represent the number of culture days; mt1 represents the cell number on Day t1; and mt2 the cell number on Day t2 (note that, t2 > t1).

Accordingly, the unit of the specific growth rate is (cells/cells/day) = (1/day).

From the viewpoint of obtaining cell number required for manufacture of a pharmaceutical composition at low cost for a short term, the specific growth rate of a cell population comprising adherent stem cells in the present invention is preferably 0.40 (1/day) or more, more preferably 0.41 (1/day) or more, further preferably 0.42 (1/day) or more, further preferably 0.43 (1/day) or more, further preferably 0.44 (1/day) or more, further preferably 0.45 (1/day) or more, further preferably 0.46 (1/day) or more, further preferably 0.48 (1/day) or more, further preferably 0.50 (1/day) or more, further preferably 0.52 (1/day) or more, further preferably 0.54 (1/day) or more, further preferably 0.55 (1/day) or more, further preferably 0.56 (1/day) or more, further preferably 0.58 (1/day) or more, further preferably 0.60 (1/day) or more, further preferably 0.62 (1/day) or more, further preferably 0.64 (1/day) or more, further preferably 0.66 (1/day) or more, further preferably 0.68 (1/day) or more, further preferably 0.70 (1/day) or more, further preferably 0.72 (1/day) or more, further preferably 0.74 (1/day) or more, further preferably 0.76 (1/day) or more, further preferably 0.78 (1/day) or more, further preferably 0.80 (1/day) or more, further preferably 0.82 (1/day) or more, further preferably 0.84 (1/day) or more, further preferably 0.86 (1/day) or more, and particularly preferably 0.87 (1/day) or more. The upper limit of the specific growth rate of a cell population comprising adherent stem cells in the present invention is not particularly limited, and is, for example, 1.0 (1/day) or less, or 0.90 (1/day) or less.

In a cell population according to the present invention, the adherent stem cells can be cultured up to preferably Day 20 or later, further preferably Day 25 or later, Day 30 or later, Day 35 or later, Day 40 or later, Day 45 or later, Day 50 or later, Day 55 or later, Day 60 or later, Day 65 or later, Day 70 or later, Day 75 or later, Day 80 or later, Day 85 or later, Day 90 or later, Day 95 or later, Day 100 or later, Day 105 or later, or Day 110 or later, after the start of *in vitro* culture.

The possible number of passages of a cell population comprising adherent stem cells and provided by the present invention is 1 or more, preferably 2 or more, more preferably 3 or more, further preferably 4 or more, further preferably 5 or more, further preferably 6 or more, further preferably 8 or more, further preferably 10 or more, further preferably 12 or more, further preferably 14 or more, further preferably 16 or more, further preferably 18 or more, further preferably 20 or more, further preferably 22 or more, further preferably 24 or more and further preferably 25 or more. The upper limit of possible passage number is not particularly limited and is, for example, 50 or less, 45 or less, 40 or less, 35 or less or 30 or less.

The cell population comprising adherent stem cells provided by the present invention may be passaged. The lower limit of the number of passages is preferably 1 or more, more preferably 2 or more, further preferably 3 or more, further preferably 4 or more, and further preferably 5 or more. In addition, the upper limit of the number of passages is preferably 25 or less, further preferably 20 or less, further preferably 15 or less, and further preferably 10 or less.

The cell population comprising adherent stem cells and provided by the present invention can be subjected to population doubling preferably 10 times or more, further preferably 20 times or more, 30 times or more, 40 times or more, 50 times or more, or 60 times or more. The cell population comprising adherent stem cells and provided by the present invention can be subjected to population doubling; for example, 100 times or less, 90 times or less, 80 times or less or 70 times or less; but are not limited to these.

Population doubling may be carried out on the cell population comprising adherent stem cells and provided by the present invention. The lower limit of the number of population doubling is preferably 5 or more, further preferably 10 or more, further preferably 15 or more, further preferably 20 or more, further preferably 25 or more, and further preferably 30 or more. In addition, the upper limit of the number of population doubling is preferably 60 or less, further preferably 55 or less, and further preferably 50 or less.

The number of population doubling is a number of times of division of cell population in a certain culture period and is calculated according to an equation: $[\log_{10}(\text{cell number at the completion of culture}) - \log_{10}(\text{cell number at the start of culture})] / \log_{10}(2)$. In a case where subculture is performed, the number of population doubling for each passage is calculated according to the equation described above and then cumulated, and thereby a total number of population doubling is calculated.

The immunosuppressive activity of the cell population comprising adherent stem cells and provided by the present invention can be evaluated by the expression level of TNFAIP6 (tumor necrosis factor, alpha-induced protein 6) gene, the proliferation inhibition rate of human peripheral blood mononuclear cells (hPBMCs) in the mixed lymphocyte reaction test (MLR test), and/or animal experimentation.

The sequence of TNFAIP6 (tumor necrosis factor, alpha-induced protein 6) gene is registered as ID: 7130 in the gene database of the National Center for Biotechnology Information. TNFAIP6 is a gene consisting of the nucleotide sequence represented by SEQ ID NO: 13 or a gene encoding a polypeptide consisting of the amino acid sequence represented by SEQ ID NO: 14.

The expression level of TNFAIP6 gene can be evaluated, for example, by a microarray, RT-PCR, quantitative RT-PCR or northern blot hybridization, but are not limited to these.

As a method for measuring the relative expression level of TNFAIP6 gene to the expression level of SDHA gene, a microarray can be used. Microarray can be performed specifically by the procedures described above.

The lower limit of the relative expression level of TNFAIP6 gene to the expression level of SDHA gene may be 0.5 or more, 0.6 or more, 0.7 or more, 0.8 or more, 0.9 or more, 1.0 or more, 1.1 or more, 1.2 or more, 1.3 or more, 1.4 or more, 1.5 or more, 1.6 or more, 1.7 or more, 1.8 or more, 1.9 or more or 2.0 or more. The upper limit of the relative expression level of TNFAIP6 gene to the expression level of SDHA gene is not particularly limited and is, for example, 4.0 or less, 3.0 or less, or 2.0 or less.

The proliferation inhibition rate of human peripheral blood mononuclear cells (hPBMCs) in the mixed lymphocyte reaction test (MLR test) can be evaluated by the following procedures (1) to (6):

(1) a target cell population is seeded in a 96-well plate for adhesion culture at $2 \times 10^4$ cells/well and adherent-cultured in $\alpha$ MEM comprising 10% (final concentration) fetal bovine serum (FBS) (inactivated) for 24 hours.

(2) To the cell population adherent-cultured for 24 hours, 10 $\mu$g/mL (final concentration) of mitomycin C is added and incubated at 37°C for 2 hours.

(3) As a co-culture group, hPBMCs are seeded at $2 \times 10^5$ cells/well in the cell population comprising mitomycin C. As a single culture group (control group), hPBMCs are seeded at $2 \times 10^5$ cells/well to a well not comprising the cell population. Individual groups (co-culture group, single culture group), to which 2.5 $\mu$g/mL (final concentration) phytohemagglutinin (PHA) is added, are cultured at 37°C for 96 hours.

(4) After culture for 96 hours, the supernatant comprising hPBMCs is collected and the total cell number and the dead cell number of hPBMCs are determined by an automatic cell counter, NucleoCounter (manufactured by ChemoMetec).

(5) Viable cell number of hPBMCs is calculated by the following equation.

$$\text{Viable cell number of hPBMCs} = \text{total cell number of hPBMCs} - \text{dead cell number of hPBMCs}$$

(6) The proliferation inhibition rate of hPBMCs is calculated by the following equation:

$$\text{The proliferation inhibition rate of hPBMCs (\%)} = 100 - (\text{viable cell number of hPBMCs in co-culture group}/\text{viable cell number of hPBMCs in single culture group}) \times 100$$

The timing for evaluating the immunosuppressive activity described above is not particularly limited, and examples thereof include immediately after cells are separated from a biological sample, during a culture step, after purification in the culture step, immediately after n times passages (n represents an integer of 1 or more), during maintenance culture, before cryopreservation, after thawing and before formulation as a pharmaceutical composition.

The cell viability of the cell population comprising adherent stem cells and provided by the present invention can be determined, for example, by trypan blue staining or MTT (3-(4,5-Dimethyl-2-thiazolyl)-2,5-diphenyltetrazolium Bromide) assay, but is not limited to these.

The cell viability of the cell population comprising adherent stem cells and provided by the present invention is preferably 70% or more, further preferably 75% or more, 80% or more, 85% or more, 90% or more, 91% or more, 92% or more, 93% or more, 94% or more, 95% or more, 96% or more, 97% or more, 98% or more, 99% or more, or 100%.

The origin of adherent stem cells is not particularly limited, and adherent stem cells derived from, for example, a fetal appendage, bone marrow, adipose or tooth pulp can be used. The adherent stem cells are preferably adherent stem cells derived from a fetal appendage, and more preferably adherent stem cells derived from the amnion. The adherent stem cells are adherent stem cells isolated from an autologous, allogeneic or heterologous biological sample, and preferably adherent stem cells isolated from an allogeneic biological sample.

The adherent stem cells are recombinant or non-recombinant adherent stem cells, and preferably non-recombinant adherent stem cells.

The cell population according to the present invention may comprise any number of adherent stem cells. The cell population according to the present invention can include, but are not limited to, not less or not more than $1.0 \times 10^1$ cells, $1.0 \times 10^2$ cells, $1.0 \times 10^3$ cells, $1.0 \times 10^4$ cells, $1.0 \times 10^5$ cells, $1.0 \times 10^6$ cells, $1.0 \times 10^7$ cells, $1.0 \times 10^8$ cells, $1.0 \times 10^9$ cells, $1.0 \times 10^{10}$ cells, $1.0 \times 10^{11}$ cells, $1.0 \times 10^{12}$ cells and $1.0 \times 10^{13}$ cells.

The cell population according to the present invention may comprise any component in addition to adherent stem cells. Examples of such a component can include, but are not limited to, salts, polysaccharides (e.g., hydroxyethyl starch (HES) and dextran), proteins (e.g., albumin), dimethyl sulfoxide (DMSO), amino acids, and medium components (e.g., components comprised in RPMI1640 medium).

The cell population according to the present invention may be preserved in a frozen state until immediately before use. The cell population described above may comprise cryopreservation solution in addition to adherent stem cells. As the cryopreservation solution described above, a commercially available cryopreservation solution may be used. Examples thereof include, but are not limited to, CP-1 (registered trademark) (manufactured by Kyokuto Pharmaceutical Industrial Co, Ltd.), BAMBANKER (manufactured by Lymphotec Inc.), STEM-CELLBANKER (manufactured by Nippon Zenyaku Kogyo Co., Ltd.), ReproCryo RM (manufactured by REPROCELL Inc.), CryoNovo (manufactured by Akron Biotechnology, LLC.), MSC Freezing Solution (manufactured by Biological Industries Inc.), and CryoStor (manufactured by HemaCare Inc.).

The cell population according to the present invention may be provided as a composition in combination with a vehicle. As the vehicle, preferably a liquid vehicle (e.g., media, dimethyl sulfoxide (DMSO), cryopreservation solutions or pharmaceutically acceptable vehicles described below) can be used.

The composition comprising the cell population according to the present invention and the vehicle may be in any cell concentration. Exemplary cell concentrations of the composition comprising the cell population according to the present invention and a vehicle include, but are not limited to, not less or not more than $1.0 \times 10^1$ cells/mL, $1.0 \times 10^2$ cells/mL, $1.0 \times 10^3$ cells/mL, $1.0 \times 10^4$ cells/mL, $1.0 \times 10^5$ cells/mL, $1.0 \times 10^6$ cells/mL, $1.0 \times 10^7$ cells/mL, $1.0 \times 10^8$ cells/mL, $1.0 \times 10^9$ cells/mL, or $1.0 \times 10^{10}$ cells/mL.

[3] Production Method a cell population comprising adherent stem cells

The production method of a cell population comprising adherent stem cells according to the present invention is a method comprising obtaining a cell population having the following cell characteristics (a) and (b):

    (a) the cell population is positive for expressions of PLIN2 gene and NEFM gene, and
    (b) the cell population satisfies the relative expression level of PLIN2 gene to the expression level of SDHA gene of 1.50 or more.

In other words, the production method of a cell population comprising adherent stem cells according to the present invention is a method comprising a step of preparing a cell population comprising adherent stem cells under such a condition that the cell characteristics (a) and (b) described above are maintained. The conditions (a) and (b) described above serve as indices for formation of a cell population comprising adherent stem cells exhibiting high immunosuppressive activity and/or proliferative property; and the production method according to the present invention is not particularly limited as long as the indices are satisfied.

The production method according to the present invention may comprise a cell population obtainment step of obtaining a cell population comprising adherent stem cells by enzymatically treating a biological sample (for example, a fetal appendage such as the amnion) comprising adherent stem cells. Said cell population obtainment step may be a step comprising a step of obtaining the amnion by cesarean section. Said cell population obtainment step may comprise a step of washing a biological sample comprising adherent stem cells.

The amnion consists of an epithelial cell layer and an extracellular matrix layer, and the latter layer comprises amniotic adherent stem cells. Like other epithelial cells, the amniotic epithelial cells are characterized by expression of an epithelial adhesion factor (EpCAM CD326). On the other hand, the amniotic adherent stem cells do not express such epithelial-specific surface antigen of CD326 and they can be easily distinguished by flow cytometry.

The cell population comprising adherent stem cells according to the present invention is preferably a cell population obtained by treating a sample comprising an epithelial cell layer and an extracellular matrix layer collected from a fetal appendage with at least collagenase.

The enzymatic treatment of a sample collected from a fetal appendage (preferably a sample comprising an epithelial cell layer and an extracellular matrix layer) is preferably a treatment with an enzyme (or a combination of enzymes) that can release adherent stem cells comprised in the extracellular matrix layer of the fetal appendage, but does not degrade the epithelial cell layer. Examples of the enzyme described above can include, but are not particularly limited to, collagenase and/or metalloproteinase. Examples of the metalloproteinase can include, but are not particularly limited to, thermolysin and/or dispase, which is metalloproteinase that cleaves nonpolar amino acids at their N-terminal sides.

The active concentration of the collagenase is preferably 50 PU/ml or higher, more preferably 100 PU/ml or higher, and further preferably 200 PU/ml or higher. The active concentration of the collagenase is, but is not particularly limited to, for example, 1000 PU/ml or lower, 900 PU/ml or lower, 800 PU/ml or lower, 700 PU/ml or lower, 600 PU/ml or lower, or 500 PU/ml or lower. In this context, PU (Protease Unit) is defined as the amount of the enzyme that degrades 1 $\mu$g of FITC-collagen in 1 minute at 30°C and pH 7.5.

The active concentration of the metalloproteinase (e.g., thermolysin and/or dispase) is preferably 50 PU/ml or higher, more preferably 100 PU/ml or higher, and further preferably 200 PU/ml or higher. Also, the active concentration of the metalloproteinase is preferably 1000 PU/ml or lower, more preferably 900 PU/ml or lower, further preferably 800 PU/ml or lower, further preferably 700 PU/ml or lower, further preferably 600 PU/ml or lower, and further preferably 500 PU/ml or lower. In this context, PU (Protease Unit) in an aspect of using dispase as the metalloproteinase is defined as the amount of the enzyme that releases an amino acid corresponding to 1 $\mu$g tyrosine from casein lactate in 1 minute at 30°C and pH 7.5. In the concentration range of the enzyme described above, adherent stem cells comprised in the extracellular matrix layer can be efficiently released while preventing contamination with epithelial cells comprised in the epithelial cell layer of the fetal appendage. The preferred combination of the concentrations of the collagenase and/or the metalloproteinase can be determined by the microscopic observation of the fetal appendage after the enzymatic treatment, or the flow cytometry of the obtained cells.

It is preferred to treat the fetal appendage at the same time with collagenase and metalloproteinase in combination, from the viewpoint of efficiently collecting live cells. In this case, thermolysin and/or dispase can be used as the metalloproteinase, though the metalloproteinase is not limited thereto. Adherent stem cells can be easily obtained by treating the fetal appendage only once with an enzyme solution comprising collagenase and metalloproteinase. The treatment at the same time can reduce the risk of contamination by bacteria, viruses, and the like.

For the enzymatic treatment of the fetal appendage, it is preferred to immerse the amnion washed using a washing solution such as physiological saline or Hank's balanced salt solution in the enzyme solution, and perform the treatment with stirring using stirring means. A stirrer or a shaker can be used as stirring means described above from the viewpoint of efficiently releasing adherent stem cells comprised in the extracellular matrix layer of the fetal appendage, though the stirring means is not limited thereto. The stirring rate is not particularly limited and is, for example, 10 rpm or more, 30 rpm or more or 50 rpm or more when using a stirrer or a shaker. Also, the stirring rate is not particularly limited and is, for example, 100 rpm or less, 80 rpm or less or 60 rpm or less when using a stirrer or a shaker. The enzymatic treatment duration is not particularly limited and is, for example, 10 minutes or longer, 30 minutes or longer, 50 minutes or longer, 70 minutes or longer or 90 minutes or longer. Also, the enzymatic treatment duration is not particularly limited and is, for example, 6 hours or shorter, 4 hours or shorter, 2 hours or shorter, or 100 minutes or shorter. The enzymatic treatment temperature is not particularly limited and is, for example, 16°C or higher, 20°C or higher, 24°C or higher, 28°C or higher, 32°C or higher or 36°C or higher. Also, the enzymatic treatment temperature is not particularly limited and is, for example, 40°C or lower, 39°C or lower, or 38°C or lower.

In the production method according to the present invention, if desired, the released adherent stem cells can be separated and/or collected from the enzyme solution comprising the released adherent stem cells by a known method such as a filter, centrifugation, a hollow fiber separation membrane, or a cell sorter. Preferably, the enzyme solution comprising the released adherent stem cells is filtered through a filter. In an aspect of filtering the enzyme solution through a filter, only the released cells pass through the filter, whereas an undegraded epithelial cell layer remains on the filter without passing through the filter. Therefore, not only can the released adherent stem cells be easily separated and/or collected, but the risk of contamination by bacteria, viruses, and the like can be reduced. Examples of the filter can include, but are not particularly limited to, mesh filters. The pore size (mesh size) of the mesh filter is not particularly limited and is, for example, 40 $\mu$m or larger, 60 $\mu$m or larger, 80 $\mu$m or larger, or 90 $\mu$m or larger. Also, the pore size of the mesh filter is not particularly limited and is, for example, 200 $\mu$m or smaller, 180 $\mu$m or smaller, 160 $\mu$m or smaller, 140 $\mu$m or smaller, 120 $\mu$m or smaller, or 100 $\mu$m or smaller. The filtration rate is not particularly limited. By using the pore size of the mesh filter within the range described above, the enzyme solution comprising the adherent stem cells can be filtered by free fall. This can prevent decrease in cell survival rate.

Nylon is preferably used as a material for the mesh filter. A tube comprising a 40 $\mu$m, 70 $\mu$m, 95 $\mu$m, or 100 $\mu$m nylon mesh filter such as a Falcon cell strainer, which is widely used for research purposes, can be used. Alternatively, medical mesh cloth (nylon and polyester) used for hemodialysis and the like can be used. Further, an arterial filter used for extracorporeal circulation (polyester mesh filter, pore size: 40 $\mu$m or larger and 120 $\mu$m or smaller) can also be used. A mesh made of any other material, for example, a stainless-steel mesh filter, may also be used.

Preferably, the adherent stem cells are allowed to pass through a filter in natural drop (free fall). It is also possible to force the cells to pass through a filter by suction using a pump or the like. In this case, minimum necessary pressurization is desirable in order to avoid damage of the cells.

The adherent stem cells that have passed through the filter can be collected by centrifugation after dilution of the filtrate with two times or more its volume of a medium or balanced salt buffer solution. Examples of the balanced salt buffer solution that can be used include, but are not limited to, physiological saline, Dulbecco's phosphate buffer (DPBS), Earle's balanced salt solution (EBSS), Hank's balanced salt solution (HBSS), and phosphate buffer (PBS).

The cell population obtained by the cell population obtainment step described above is prepared under the conditions: (a) the cell population is positive for expressions of PLIN2 gene and NEFM gene and (b) the cell population satisfies the relative expression level of PLIN2 gene to the expression level of SDHA gene of 1.50 or more. The conditions are useful as indices for obtaining a cell population comprising adherent stem cells exhibiting high immunosuppressive activity and/or proliferative property. The preparation method is not particularly limited as long as the indices are satisfied. Examples of such a method may include: separating a cell population satisfying the above (a) by a cell sorter, and then, selecting, from the obtained cell population, a cell population satisfying the above (b); and separating a cell population satisfying the above (b), and then, selecting, from the obtained cell population, a cell population satisfying the above (a) by a cell sorter. As another preparation method for satisfying the above indices, a method of culturing a cell population under conditions satisfying the above (a) and (b) may be mentioned.

Examples of a culture method satisfying the indices include a step of repeating, a plurality of times, the seeding of the cell population into an uncoated plastic culture vessel at a density of 100 to 20,000 cells/cm$^2$ followed by culture. The lower limit of the density of the cell population for seeding is further preferably 200 cells/cm$^2$ or more, further preferably 400 cells/cm$^2$ or more, further preferably 600 cells/cm$^2$ or more, further preferably 800 cells/cm$^2$ or more, further preferably 1000 cells/cm$^2$ or more, further preferably 1200 cells/cm$^2$ or more, further preferably 1400 cells/cm$^2$ or more, further preferably 1600 cells/cm$^2$ or more, further preferably 1800 cells/cm$^2$ or more, and further preferably 2000 cells/cm$^2$ or more. The upper limit of the density of the cell population for seeding is further preferably 18000 cells/cm$^2$ or less, further preferably 16000 cells/cm$^2$ or less, further preferably 14000 cells/cm$^2$ or less, further preferably 12000 cells/cm$^2$ or less, further preferably 10000 cells/cm$^2$ or less, and further preferably 8000 cells/cm$^2$ or less.

Examples of the other culture methods that satisfy the indices include a step of repeating a plurality of times the seeding of the cell population into a plastic culture vessel coated with a coating agent at a density of 100 to 20000 cells/cm$^2$ followed by culture. Preferred density conditions for the seeding of the cell population are similar to the conditions described above.

Examples of the coating agent include, but are not limited to, extracellular matrix, fibronectin, vitronectin, osteopontin, laminin, entactin, collagen I, collagen II, collagen III, collagen IV, collagen V, collagen VI, gelatin, poly-L-ornithine, poly-D-lysine, and Matrigel (registered trademark) matrix.

The medium for use in the culture can be prepared by utilizing any liquid medium for animal cell culture as a basal medium and, if necessary, appropriately adding other components (albumin, serum, a serum replacement reagent, a growth factor, or human platelet lysate, etc.) thereto.

Examples of the basal medium that can be used include, but are not particularly limited to, media such as BME medium, BGJb medium, CMRL1066 medium, Glasgow MEM medium, improved MEM zinc option medium, IMDM medium (Iscove's modified Dulbecco's medium), Medium 199 medium, Eagle MEM medium, αMEM (alpha modification of minimum essential medium eagle) medium, DMEM medium (Dulbecco's modified Eagle's medium), Ham's F10 medium, Ham's F12 medium, RPMI 1640 medium, Fischer's medium, and mixed medium thereof (e.g., DMEM/F12 medium (Dulbecco's modified Eagle's medium/nutrient mixture F-12 Ham)).

Alternatively, the medium for use in the culture may be a commercially available serum-free medium. Examples thereof include, but are not particularly limited to, STK1 and STK2 (manufactured by DS Pharma Biomedical Co., Ltd.), EXPREP MSC Medium (manufactured by BioMimetics Sympathies Inc.), and Corning stemgro human adherent stem cell medium (manufactured by Corning Inc.).

Examples of other components to be added to the basal medium include albumin, serum, serum replacement reagents, growth factors or human platelet lysate. In an aspect of adding albumin to the basal medium, the concentration of albumin is preferably higher than 0.05% and 5% or lower. In an aspect of adding serum to the basal medium, the concentration of serum is preferably 5% or higher. In an aspect of adding a growth factor, a reagent (e.g., protein such as heparin, gel, polysaccharide) for stabilizing the growth factor in a medium may be further added in addition to the growth factor; or the growth factor stabilized in advance may be added to the basal medium. Examples of the growth factor that can be used include, but are not particularly limited to, fibroblast growth factor (FGF), epidermal growth factor (EGF), transforming growth factor (TGF), vascular endothelial cell growth factor (VEGF), platelet-derived growth factor (PDGF), and families thereof.

Examples of further other culture methods that satisfy the indices include culturing with addition of human platelet lysate (hPL) to the basal medium for use in the culture. The human platelet lysate is preferably subjected to inactivation and/or sterilization treatment for bacteria and viruses, in advance. As the above human platelet lysate, commercially available human platelet lysate may be used. Examples thereof include, but are not limited to, Stemulate (manufactured by Cook Regentec), PLTMax (manufactured by Mill Creek Life Science), UltraGRO (manufactured by AventaCell BioMedial) and PLUS (manufactured by Compass Biomedical).

The final concentration of human platelet lysate in a medium is preferably 1% or more, further preferably 2% or more, further preferably 3% or more, further preferably 4% or more and further preferably 5% or more. The final concentration of platelet lysate in a medium is preferably 20% or less, further preferably 18% or less, further preferably 16% or less, further preferably 14% or less, further preferably 12% or less, further preferably 10% or less, further preferably 9% or less, further preferably 8% or less, further preferably 7% or less and further preferably 6% or less.

Timing for adding human platelet lysate is not particularly limited and examples thereof include the beginning of a culture step, during the culture step, after purification in the culture step, immediately after n times passages (n represents an integer of 1 or more), during maintenance culture, before cryopreservation, or after thawing.

The culture of adherent stem cells can be performed by, for example, the following steps. First, a cell suspension is centrifuged; the supernatant is removed; and the obtained cell pellet is suspended in a medium. Next, the cells are seeded into a plastic culture vessel and cultured to 95% confluence or less using a medium in an environment of a $CO_2$ concentration of 3% or higher and 5% or lower at 37°C. Examples of the medium can include, but are not limited to, $\alpha$MEM, M199, and mediums using these as a base. The cells obtained by the culture as described above are cells cultured once.

Examples of the culture period of a single culture process can include 2 to 15 days and specifically 2 days, 3 days, 4 days, 5 days, 6 days, 8 days, 10 days, 12 days, 14 days and 15 days.

The cells cultured once described above can be further passaged and cultured, for example, as follows: first, the cells cultured once are treated by cell dissociation means, and thereby dissociated from the plastic culture vessel. Next, the obtained cell suspension is centrifuged, the supernatant is removed, and the obtained cell pellet is suspended in a medium. Finally, the cells are seeded to a plastic culture vessel, and cultured to 95% or less confluence using a medium in an environment of a $CO_2$ concentration of 3% or higher, and 5% or lower at 37°C. Examples of the medium can include, but are not limited to, $\alpha$MEM, M199, and media based thereon. The cells obtained by the passage and the culture as described above are cells passaged once. Cells passaged n times can be obtained by similar passage and culture (n represents an integer of 1 or more). From the viewpoint of producing the cells at a large scale, the lower limit of passage number n is, for example, 1 or more, preferably 2 or more, more preferably 3 or more, further preferably 4 or more, and further preferably 5 or more. In addition, from the viewpoint of suppressing cell senescence, the upper limit of passage number n is, for example, preferably 25 or less, 20 or less, 15 or less, or 10 or less.

As the cell dissociation means, a cell dissociation agent, for example, may be used. As the cell dissociation agent, trypsin, collagenase, dispase, ethylenediaminetetraacetic acid (EDTA) or the like can be used, but the cell dissociation agent is not particularly limited. As the cell dissociation agent, a commercially available cell dissociation agent may be used. Examples thereof include, but are not limited to, trypsin-EDTA solution (manufactured by Thermo Fisher Scientific Inc.), TrypLE Select (manufactured by Thremo Fisher Scientific Inc.), Accutase (manufactured by Stemcell Technologies Inc.), and Accumax (manufactured by Stemcell Technologies Inc.). In addition, as cell dissociation means, physical cell dissociation means may be used, and examples thereof to be used include, but are not limited to, a cell scraper (manufacture by Corning Inc.). Cell dissociation means may be used alone or a plurality of cell dissociation means may be used in combination.

According to the production method in the present invention, a cell population comprising adherent stem cells exhibiting high immunosuppressive activity and/or proliferative property can be obtained. The lower limit of the cell number obtained per batch of culture (cell number obtained per unit surface area per unit number of culture days) differs depending on, e.g., the number of seeded cells, the seeding density, and is, for example, $5.0 \times 10^3$ (cells/cm$^2$/day) or more, $6.0 \times 10^3$ (cells/cm$^2$/day) or more, $8.0 \times 10^3$ (cells/cm$^2$/day) or more, $1.0 \times 10^4$ (cells/cm$^2$/day) or more, $1.1 \times 10^4$ (cells/cm$^2$/day) or more, or $1.2 \times 10^4$ (cells/cm$^2$/day) or more. Also, the upper limit of the obtained cell number per batch of culture is not particularly limited and is, for example, $1.0 \times 10^5$ (cells/cm$^2$/day) or less, $8.0 \times 10^4$ (cells/cm$^2$/day) or less, $6.0 \times 10^4$ (cells/cm$^2$/day) or less, $4.0 \times 10^4$ (cells/cm$^2$/day) or less, or $2.0 \times 10^4$ (cells/cm$^2$/day) or less.

According to the production method in the present invention, a cell population comprising adherent stem cells having high immunosuppressive activity and/or proliferative property can be obtained. Accordingly, the adherent stem cells obtained by the production method according to the present invention can be cultured preferably up to Day 20 or later, further preferably Day 30 or later, Day 40 or later, Day 50 or later, Day 60 or later, Day 70 or later, Day 80 or later, Day 90 or later, Day 100 or later, or Day 110 or later, after the start of *in vitro* culture.

The cell population comprising adherent stem cells obtained by the production method according to the present invention can also be cultured up to doubling number of preferably 10 or more, 20 or more, 30 or more, 40 or more, 50 or more, or 60 or more, after the start of *in vitro* culture.

The production method according to the present invention may comprise an identification step of identifying a cell population comprising adherent stem cells having high immunosuppressive activity and/or proliferative property by using, as indices, (a) the cell population is positive for expressions of PLIN2 gene and NEFM gene and (b) the cell population satisfies the relative expression level of PLIN2 gene to the expression level of SDHA gene of 1.50 or more.

Means for identifying the cell population comprising adherent stem cells is preferably a microarray and/or quantitative RT-PCR.

The relative expression levels of PLIN2 gene and/or NEFM gene to the expression level of SDHA gene can be measured using a microarray in accordance with the procedures described above.

The timing for performing the above identification is not particularly limited, and examples thereof include immediately after separation of cells from a biological sample, during a culture step, after purification in the culture step, immediately after n times passages (n represents an integer of 1 or more), during maintenance culture, before cryopreservation, after thawing, and before formulation as a pharmaceutical composition.

The production method according to the present invention may comprise a step of selectively separating the identified cell population after identifying the cell population comprising the adherent stem cells by using the above conditions (a) and (b) as indices. Examples of the means for selectively separating the identified cell population include, but are not particularly limited to, separation of a cell population by a cell sorter, and purification of a cell population by culture.

The production method according to the present invention also may comprise a step of cryopreserving the cell population comprising adherent stem cells. In an aspect of comprising a step of cryopreserving the cell population, the cell population thawed, if necessary, may be identified, separated, collected and/or cultured. Alternatively, the cell population thawed may be directly used.

Examples of the means for cryopreserving the cell population comprising adherent stem cells include, but are not particularly limited to, program freezers, deep freezers, and immersing in liquid nitrogen. In the case of using a program freezer, the temperature for freezing is preferably -30°C or lower, -40°C or lower, -50°C or lower, -80°C or lower, -90°C or lower, -100°C or lower, -150°C or lower, -180°C or lower, or -196°C (temperature of liquid nitrogen) or lower. In the case of using a program freezer, the preferable rate of freezing is, for example, -1°C/min, -2°C/min, -5°C/min, -9°C/min, -10°C/min, -11°C/min, or -15°C/min. In the case of using a program freezer as a freezing means, the temperature can be lowered to a temperature to -50°C or higher and -30°C or lower (e.g., -40°C) at a freezing rate of, for example, -2°C/min or more and -1°C/min or less, and further lowered to a temperature of - 100°C or higher and -80°C or lower (for example, -90°C) at a freezing rate of -11°C/min or more and -9°C/min or less (e.g., -10°C/min). When immersing in liquid nitrogen is used as a freezing means, the temperature can be rapidly lowered to, for example -196°C for freezing, and then, cryopreservation can be carried out in liquid nitrogen (gas phase).

For freezing by the freezing means, the cell population may be frozen in a state comprised in any preservation container. Examples of the preservation container include, but are not limited to, cryotubes, cryovials, freezing bags, and infusion bags.

For freezing by the freezing means, the cell population may be frozen in any cryopreservation solution. As the cryopreservation solution described above, a commercially available cryopreservation solution may be used. Examples of the cryopreservation solution include, but are not limited to, CP-1 (registered trademark) (manufactured by Kyokuto Pharmaceutical Industrial Co., Ltd.), BAMBANKER (manufactured by Lymphotec Inc.), STEM-CELLBANKER (manufactured by Nippon Zenyaku Kogyo Co., Ltd.), ReproCryo RM (manufactured by REPROCELL Inc.), CryoNovo (Akron Biotechnology, LLC.), MSC Freezing Solution (Biological Industries Inc.), and CryoStor (HemaCare Inc.).

The cryopreservation solution can comprise polysaccharides at a defined concentration. The preferable concentration of polysaccharides is, for example, 1% by mass or higher, 2% by mass or higher, 4% by mass or higher, or 6% by mass or higher. In addition, the preferable concentration of polysaccharides is, for example, 20% by mass or lower, 18% by mass or lower, 16% by mass or lower, 14% by mass or lower, or 13% by mass or lower. Examples of the polysaccharides include, but are not limited to, hydroxyethyl starch (HES) and dextran (e.g., Dextran40).

The cryopreservation solution can comprise dimethyl sulphoxide (DMSO) at a defined concentration. The preferable concentration of DMSO is, for example, 1% by mass or higher, 2% by mass or higher, 3% by mass or higher, 4% by mass or higher, or 5% by mass or higher. Also, the preferable concentration of DMSO is, for example, 20% by mass or lower, 18% by mass or lower, 16% by mass or lower, 14% by mass or lower, 12% by mass or lower, or 10% by mass or lower.

The cryopreservation solution may be a solution comprising albumin at a defined concentration higher than 0% by mass. The preferable concentration of albumin is, for example, 1% by mass or higher, 2% by mass or higher, 3% by mass or higher, or 4% by mass or higher. Also, the preferable concentration of albumin is, for example, 30% by mass or lower, 20% by mass or lower, 10% by mass or lower or 9% by mass or lower. Examples of albumin can include, but are not limited to, bovine serum albumin (BSA), mouse albumin, and human albumin.

The production method according to the present invention can comprise a step of washing the cell population comprising adherent stem cells. Examples of a washing solution to be used in the step of washing the cell population comprising adherent stem cells include, but are not limited to, physiological saline, Dulbecco's phosphate buffer (DPBS), Earle's balanced salt solution (EBSS), Hank's balanced salt solution (HBSS) and phosphate-buffer (PBS). Washing a cell population can reduce or remove allergen, endotoxin or the like. Examples of the allergen include, but are not limited to, bovine serum albumin (BSA), swine trypsin and swine heparin.

The production method according to the present invention can comprise, if desired, a step of removing undesirable cell aggregates from the cell population comprising adherent stem cells. The step of removing undesirable cell aggregates from the cell population comprising adherent stem cells may be a step comprising a step of filtering a cell population comprising adherent stem cells (cell suspension) by a filter.

The production method according to the present invention can comprise a step of filling the cell population comprising adherent stem cells into a preservation container. Examples of such a preservation container include, but are not limited to, cryotubes, cryovials, freezing bags, and infusion bags.

[4] Method for monitoring the immunosuppressive activity and/or proliferative property of adherent stem cells, method for evaluating a donor and/or a biological sample collected from the donor, and method for determining and/or predicting an optimal enzymatic treatment condition

In the present invention, in a cell population comprising adherent stem cells, the immunosuppressive activity and/or proliferative property of adherent stem cells can be monitored by performing measurement (preferably by performing measurement over time) using, as indices, conditions: (a) the cell population is positive for expressions of PLIN2 gene and NEFM gene and (b) the cell population satisfies the relative expression level of PLIN2 gene to the expression level of SDHA gene of 1.50 or more. Examples of the step that requires monitoring include a culture step, a cryopreservation step and/or a formulation step.

In the culture step, for example, measurement of the indices over time allows changes in the immunosuppressive activity and/or proliferative property of adherent stem cells to be quickly and easily known and predicted. It can be known that, in a cell population comprising adherent stem cells satisfying the indices, the adherent stem cells maintain high immunosuppressive activity and/or proliferative property. On the other hand, when a culture state continues with a value deviating from the indices, it can be predicted that the immunosuppressive activity and/or proliferative property of adherent stem cells is deteriorating. When it is read out from the indices that the immunosuppressive activity and/or proliferative property of adherent stem cells is deteriorating, deterioration of the immunosuppressive activity and/or proliferative property of adherent stem cells can be suppressed by properly changing culture conditions (change of an seeding density, a medium, a growth factor, serum, etc.) as needed. When the indices are not satisfied, only a cell population comprising adherent stem cells that satisfies the indices is separated by use of, for example, a cell sorting technique, and adherent stem cells in the cell population are seeded again and subcultured, and thereby deterioration of the immunosuppressive activity and/or proliferative property of adherent stem cells can be suppressed. At the early stage of culture, culture conditions (change of an seeding density, a medium, a growth factor, serum, etc.) may be designed such that the indices are satisfied at the final stage of the step, and thus, the indices may be satisfied at least at the final stage.

In the present invention, the quality of a donor itself and/or a biological sample collected from the donor can be evaluated by obtaining a cell population comprising adherent stem cells from the donor, determining the presence or absence of expressions of PLIN2 gene and NEFM gene and measuring the relative expression level of PLIN2 gene to the expression level of SDHA gene, and performing the measurement using, as indices, conditions: expressions of PLIN2 gene and NEFM gene are positive, (b) the cell population satisfies the relative expression level of PLIN2 gene to the expression level of SDHA gene of 1.50 or more. When a cell population comprising adherent stem cells that satisfies the indices is obtained (preferably, easily obtained), the quality of the donor and/or a biological sample collected from the donor can be confirmed to be good from the viewpoint of high immunosuppressive capacity and/or high proliferation capacity. On the other hand, the case where the relative expression level in the cell population comprising adherent stem cells deviates from the indices means that the quality of biological sample collected from the donor is bad. In this case, deterioration of the immunosuppressive activity and/or proliferative property of adherent stem cells can be suppressed by properly changing culture conditions (change of an seeding density, a medium, a growth factor, serum, etc.). When the relative expression level in the cell population comprising adherent stem cells deviates from the indices, deterioration of the immunosuppressive activity and/or proliferative property of adherent stem cells can be suppressed by separating a cell population comprising adherent stem cells satisfying the indices by use of, for example, a cell sorting technique, and seeding and culturing the adherent stem cells in the cell population. At the early stage of culture, culture conditions (change of an seeding density, a medium, a growth factor, serum, etc.) may be designed such that the indices are satisfied at the final stage of the step, and thus, the indices may be satisfied at least at the final stage. Note that, in confirming the quality of a biological sample collected from a donor, a method for preparing and treating a biological sample, and a method for culturing a cell population are not particularly limited, and any methods can be employed.

In the present invention, in regard to a cell population obtained by enzymatically treating a biological sample collected from a donor, an optimal enzymatic treatment condition can be determined and/or predicted by determining the presence or absence of expressions of PLIN2 gene and NEFM gene, and measuring the relative expression level of PLIN2 gene to the expression level of SDHA gene, and evaluating by using, as indices, conditions: expressions of PLIN2 gene and NEFM gene are positive and (b) the cell population satisfies the relative expression level of PLIN2 gene to the expression level of SDHA gene of 1.50 or more. When a cell population comprising adherent stem cells that satisfies the indices is obtained (preferably, easily obtained), the enzymatic treatment method can be determined and/or predicted to be appropriate for the biological sample collected from a donor. On the other hand, when a culture state continues with a value deviating from the indices, the enzymatic treatment method can be determined and/or predicted to be inappropriate for the biological sample collected from a donor. Note that, in determining and/or predicting an optimal enzymatic treatment method, a method for preparing and treating the biological sample, and a method for culturing a cell population are not particularly limited, and any methods can be employed.

The indices can be measured at a necessary timing. Examples of the timing include, but are not particularly limited to, immediately after separation of cells from a biological sample, during a culture step, after purification in the culture step, immediately after n times passages (n represents an integer of 1 or more), during maintenance culture, before cryopreservation, after thawing, or before formulation as a pharmaceutical composition.

[5] Pharmaceutical composition

The cell population comprising adherent stem cells according to the present invention can be used as a pharmaceutical composition. That is, the present invention provides a pharmaceutical composition comprising the cell population according to the present invention and a pharmaceutically acceptable vehicle. According to the present invention, a pharmaceutical composition comprising a cell population comprising adherent stem cells according to the present invention and additional cells that can be administered is further provided.

The pharmaceutical composition according to the present invention can be used as a cell therapy agent, for example, a therapeutic agent for intractable diseases.

The pharmaceutical composition according to the present invention can be used as a therapeutic agent for an immune-related disease. The above immune-related diseases can be treated by administering the pharmaceutical composition according to the present invention to a treatment site in an amount whereby an effect can be measured.

The present invention provides the cell population comprising adherent stem cells according to the present invention for use in a pharmaceutical composition.

The present invention provides the cell population comprising adherent stem cells according to the present invention for use in a cell therapy agent.

The present invention provides the cell population comprising adherent stem cells according to the present invention for use in an immune-related disease.

The present invention provides the cell population comprising adherent stem cells according to the present invention for use in suppression of immune response by administering to a patient or subject.

The present invention provides a method for transplanting cells to a patient or subject and a method for treating a disease of a patient or subject, comprising a step of administering a therapeutically effective amount of the cell population comprising adherent stem cells according to the present invention to the patient or the subject.

The present invention provides use of the cell population comprising adherent stem cells according to the present invention for the manufacture of a pharmaceutical composition.

The present invention provides use of the cell population comprising adherent stem cells according to the present invention for the manufacture of a cell therapy agent.

The present invention provides use of the cell population comprising adherent stem cells according to the present invention for the manufacture of a therapeutic agent for an immune-related disease.

The present invention provides use of the cell population comprising adherent stem cells according to the present invention for the manufacture of a therapeutic agent required for suppressing immune response by administration to a patient or subject.

The pharmaceutical composition according to the present invention may be obtained by diluting a cell population comprising adherent stem cells with a pharmaceutically acceptable vehicle. The pharmaceutically acceptable vehicle is not particularly limited as long as it is a solution that can be administered to a patient or a subject. The pharmaceutically acceptable vehicle may be an infusion preparation, and examples thereof include, but are not limited to, water for injection, physiological saline, 5% glucose solution, Ringer's solution, lactated Ringer's solution, acetated Ringer's solution, bicarbonated Ringer's solution, amino acid solution, starter solution (Solution I), rehydration solution (Solution II), maintenance infusion (Solution III), postoperative recovery solution (Solution IV), and Plasma-Lyte A (registered trademark).

The "patient or subject" used herein is typically a human and may be other animals. Examples of other animals include, but are not limited to, mammals such as dogs, cats, cattle, horses, pigs, goats, sheep, monkeys (cynomolgus monkey, rhesus monkey, common marmoset and Japanese monkey), ferrets, rabbits and rodents (mouse, rat, Mongolian gerbil, guinea pig and hamster); birds such as chickens and quails.

The "immune-related disease" used herein is not particularly limited as long as it is a disease related to an immune responses of a patient or subject. Examples thereof include graft-versus-host disease (GVHD), inflammatory bowel diseases (IBD), Crohn's disease, ulcerative colitis, radiation enteritis, diabetes mellitus, systemic lupus erythematosus, collagen disease, mycosis fungoides, multiple sclerosis, psoriasis, autoimmune bullous disease, and rheumatoid arthritis.

The term "treatment" used herein refers to significantly improving at least one of, for example, life prognosis, functional prognosis, viability, weight loss, anemia, diarrhea, melena, pain in the abdomen, fever, reduction in appetite, malnutrition, vomiting, fatigue, rash, inflammation, ulcer, erosion, fistula, constriction, ileus, internal bleeding, rectal bleeding, convulsion, pain, hypohepatia, and blood test items of a patient or subject, but examples are not limited to these.

The pharmaceutical composition according to the present invention may comprise any component for use in the treatment of a patient or subject. Examples of the component include, but are not limited to, salts (e.g., physiological saline, Ringer's solution, BICANATE infusion), polysaccharides (e.g., hydroxyethyl starch (HES) and dextran), proteins (e.g., albumin), dimethyl sulphoxide (DMSO), amino acids, and medium components (e.g., components comprised in RPMI1640 medium).

The pharmaceutical composition according to the present invention may comprise various additives for increasing the preservation stability, the isotonicity, the absorbability, and/or the viscosity, such as an emulsifier, a dispersant, a buffer, a preservative, a wetting agent, an antioxidant, a chelating agent, a thickener, a gelling agent and a pH adjuster. Examples of the thickener include, but are not limited to, HES, dextran, methylcellulose, xanthan gum, carboxymethylcellulose and hydroxypropyl methylcellulose. The concentration of the thickener can be optionally set according to the selected thickener, within the range of concentration that is safe when administered to the patient or the subject and achieves the desired viscosity.

The pharmaceutical composition according to the present invention may compris one or more additional medicines besides the adherent stem cells. Examples of the additional medicines include, but are not limited to, antibiotics, albumin preparations, vitamin preparations and anti-inflammatory agents. Examples of the anti-inflammatory agents include, but are not limited to, 5-aminosalicylic acid preparations, steroid preparations, immunosuppressants and biological preparations. Examples of the 5-aminosalicylic acid preparations include, but are not limited to, salazosulfapyridine and mesalazine. Examples of the steroid preparations include, but are not limited to, cortisone, prednisolone and methylprednisolone. Examples of the immunosuppressants include, but are not limited to, tacrolimus, cyclosporine, methotrexate, azathioprine and 6-mercaptopurine. Examples of the biological preparations include, but are not limited to, infliximab, adalimumab, ustekinumab, secukinumab, ixekizumab, brodalumab, tocilizumab, vedolizumab, filgotinib, golimumab, certolizumab pegol, abatacept and etanercept. The additional medicines may be other types of cells.

The additional medicines may be additional cells that can be administered. Examples of the additional cells that can be administered include, but are not limited to, blood-derived cells (e.g., white blood cells, red blood cells, mononuclear cells), vascular endothelial cells, endothelial precursor cells, vascular wall cells, pericytes, fibroblasts, skeletal myoblasts, epithelial cells, stromal cells, mature adipose cells, parenchymal cells of organs (e.g., liver parenchymal cells, cardiomyocytes) and nerve cells.

The pH of the pharmaceutical composition according to the present invention can be around neutral pH, for example, pH5.5 or more, pH6.0 or more, pH6.5 or more or pH7.0 or more and also pH10.5 or less, pH9.5 or less, pH8.5 or less or pH8.0 or less; however, the pH is not limited to these.

The cell concentration of the pharmaceutical composition according to the present invention is the concentration of cells that allows a patient or subject to whom the pharmaceutical composition has been administered to obtain therapeutic effects on diseases, compared with a patient or subject to whom the pharmaceutical composition has not been administered. The specific cell concentration can be appropriately determined depending on the form of administration, the administration method, the intended use, the age, body weight, symptoms of a patient or subject, and the like. The lower limit of cell concentration of the pharmaceutical composition according to the present invention is not particularly limited, and is, for example, $1.0 \times 10^5$ cells/mL or more, $1.0 \times 10^6$ cells/mL or more, $1.2 \times 10^6$ cells/mL or more, $1.4 \times 10^6$ cells/mL or more, $1.6 \times 10^6$ cells/mL or more, $1.8 \times 10^6$ cells/mL or more, $2.0 \times 10^6$ cells/mL or more, $3.0 \times 10^6$ cells/mL or more, $4.0 \times 10^6$ cells/mL or more, $5.0 \times 10^6$ cells/mL or more, $6.0 \times 10^6$ cells/mL or more, $7.0 \times 10^6$ cells/mL or more, $8.0 \times 10^6$ cells/mL or more, $9.0 \times 10^6$ cells/mL or more, $9.5 \times 10^6$ cells/mL or more, or $1.0 \times 10^7$ cells/mL or more. The upper limit of cell concentration of the pharmaceutical composition according to the present invention is not particularly limited, and is, for example, $1.0 \times 10^{10}$ cells/mL or less, $1.0 \times 10^9$ cells/mL or less, $8.0 \times 10^8$ cells/mL or less, $6.0 \times 10^8$ cells/mL or less, $4.0 \times 10^8$ cells/mL or less, $2.0 \times 10^8$ cells/mL or less, or $1.0 \times 10^8$ cells/mL or less.

The pharmaceutical composition according to the present invention is preferably a liquid preparation, and more preferably an injectable liquid preparation. As the injectable liquid preparation, liquid preparations suitable for injection are known in, for example, International Publication No. WO 2011/043136 and JP Patent Publication (Kokai) No. 2013-256510. The pharmaceutical composition according to the present invention may also be an injectable liquid preparation described in the above literatures.

The above liquid preparation may be a cell suspension or a liquid preparation having cells dispersed in liquid. Further, the form of cells comprised in the liquid preparation is not particularly limited, and, for example, may be single cells or cell aggregates.

If the pharmaceutical composition according to the present invention is an injectable liquid preparation, the lower limit of cell concentration of the injectable liquid preparation, from the viewpoint of enhancing therapeutic effect on a disease, is preferably $1.0 \times 10^6$ cells/mL or more, $1.2 \times 10^6$ cells/mL or more, $1.4 \times 10^6$ cells/mL or more, $1.6 \times 10^6$ cells/mL or more, $1.8 \times 10^6$ cells/mL or more, $2.0 \times 10^6$ cells/mL or more, $3.0 \times 10^6$ cells/mL or more, $4.0 \times 10^6$ cells/mL or more, $5.0 \times 10^6$ cells/mL or more, $6.0 \times 10^6$ cells/mL or more, $7.0 \times 10^6$ cells/mL or more, $8.0 \times 10^6$ cells/mL or more, $9.0 \times 10^6$ cells/mL or more, $9.5 \times 10^6$ cells/mL or more, or $1.0 \times 10^7$ cells/mL or more. In contrast, the upper limit of cell concentration of the injectable liquid preparation, from the viewpoint of simplifying preparation and administration of the injectable liquid preparation, is preferably $1.0 \times 10^9$ cells/mL or less, $8.0 \times 10^8$ cells/mL or less, $6.0 \times 10^8$ cells/mL or less, $4.0 \times 10^8$ cells/mL or less, $2.0 \times 10^8$ cells/mL or less, or $1.0 \times 10^8$ cells/mL or less.

According to one aspect of the present invention, the pharmaceutical composition according to the present invention may be a transplant preparation of a cell aggregate or sheet-like structure. As the transplant preparation of a cell aggregate, a transplant preparation comprising a cell clump, which is obtained by bonding discrete cells with an adhesive (for example, fibrinogen) is known in, for example, International Publication No. WO 2017/126549. As the sheet-like transplant preparation, a cell sheet obtained by culturing in a temperature-responsive culture dish (for example, UpCell (registered trademark) (manufactured by CellSeed Inc.)), a laminate of a sheet-like cell culture and fibrin gel, and a cell-coated sheet obtained by applying a cell suspension to a sheet-like substrate, are known in, for example, International Publication No. WO 2006/080434 and JP Patent Publication No.2016-52272. The pharmaceutical composition according to the present invention may be used for preparing various transplant preparations of a cell aggregate or sheet-like structure by employing a method described, for example, in the above literatures.

According to one aspect in the present invention, the pharmaceutical composition according to the present invention may be a gel preparation prepared by mixing the cells with a gel. As the gel preparation, for example, a cell therapy agent comprising an adherent stem cell-hydrogel composition as an active ingredient is known in JP Patent Publication (Kohyo) No. 2017-529362. The pharmaceutical composition according to the present invention can be used as gel preparations described in the above literature.

Examples of the administration method of the pharmaceutical composition according to the present invention include, but are not particularly limited to, subcutaneous injection, intradermal injection, intramuscular injection, intra-lymph nodal injection, intravenous injection, intra-arterial injection, intraperitoneal injection, intrathoracic injection, direct localized injection, direct patch and direct localized transplantation. According to an aspect of the present invention, an injectable liquid preparation can be filled in a syringe and administered through a needle or a catheter into the vein, artery, myocardium, internodal space, hepatic artery, muscle, epidural site, gum, ventricle, subcutaneous site, intradermal site, intraperitoneal site and portal vein; however, injection sites are not limited to these. Regarding the administration method of the pharmaceutical composition according to the present invention, intravenous injection, intravenous drip injection, local direct injection, local direct transplantation and others are known in, for example, JP Patent Publication (Kokai) No. 2015-61520, Onken JE, et al., American College of Gastroenterology Conference 2006 Las Vegas, NV, Abstract 121., and Garcia-Olmo D, et al., Dis Colon Rectum 2005; 48: 1416-23. The pharmaceutical composition according to the present invention can also be administered by various methods described in the above literatures.

The dose of the pharmaceutical composition according to the present invention is the amount of cells that allows a patient or subject to whom the pharmaceutical composition has been administered to obtain therapeutic effects on diseases, compared with a patient or a subject to whom the pharmaceutical composition has not been administered. A specific dose can be appropriately determined depending on the form of administration, the administration method, the intended use, the age, body weight, symptoms of a patient or subject, and the like. A single dose of adherent stem cells to a human is not particularly limited and is, for example, $1 \times 10^4$ cells/kg body weight or more, $1 \times 10^5$ cells/kg body weight or more, $5 \times 10^5$ cells/kg body weight or more, $1 \times 10^6$ cells/kg body weight or more, $2 \times 10^6$ cells/kg body weight or more, $4 \times 10^6$ cells/kg body weight or more, $6 \times 10^6$ cells/kg body weight or more, or $8 \times 10^6$ cells/kg body weight or more. A single dose of adherent stem cells to a human is not particularly limited and is, for example, $1 \times 10^{12}$ cells/kg body weight or less, $1 \times 10^{11}$ cells/kg body weight or less, $1 \times 10^{10}$ cells/kg body weight or less, $1 \times 10^9$ cells/kg body weight or less, $5 \times 10^8$ cells/kg body weight or less, $1 \times 10^8$ cells/kg body weight or less, $8 \times 10^7$ cells/kg body weight or less, $6 \times 10^7$ cells/kg body weight or less, $4 \times 10^7$ cells/kg body weight or less, or $2 \times 10^7$ cells/kg body weight or less.

When the pharmaceutical composition according to the present invention is an injectable liquid preparation, a single dose of adherent stem cells of the injectable liquid preparation to a human, from the viewpoint of enhancing therapeutic effect on a disease, is preferably, $1 \times 10^5$ cells/kg body weight or more, $5 \times 10^5$ cells/kg body weight or more, $1 \times 10^6$ cells/kg body weight or more, $2 \times 10^6$ cells/kg body weight or more, $4 \times 10^6$ cells/kg body weight or more, $6 \times 10^6$ cells/kg body weight or more, or $8 \times 10^6$ cells/kg body weight or more. Also, a single dose of adherent stem cells of the injectable liquid preparation to a human, from the viewpoint of simplifying preparation and administration of an injectable liquid preparation, is preferably, $1 \times 10^9$ cells/kg body weight or less, $5 \times 10^8$ cells/kg body weight or less, $1 \times 10^8$ cells/kg body weight or less, $8 \times 10^7$ cells/kg body weight or less, $6 \times 10^7$ cells/kg body weight or less, $4 \times 10^7$ cells/kg body weight or less, or $2 \times 10^7$ cells/kg body weight or less.

The frequency of administration of the pharmaceutical composition according to the present invention is the frequency that allows a patient or subject to whom the pharmaceutical composition has been administered to obtain therapeutic effects on diseases. A specific frequency of administration can be appropriately determined depending on the form of administration, the administration method, the intended use, the age, body weight, symptoms of a patient or subject, and the like, and is, for example, once every 4 weeks, every 3 weeks, every two weeks, every week, twice per week, three times per week, four times per week, five times per week, six times per week, or seven times per week.

The administration period of the pharmaceutical composition according to the present invention is the period that allows a patient or subject to whom the pharmaceutical composition has been administered to obtain therapeutic effects on diseases. Specific administration period can be appropriately determined depending on the form of administration, the administration method, the intended use, the age, body weight, symptoms of a patient or subject, and the like, and is, for example, a week, 2 weeks, 3 weeks, 4 weeks, 5 weeks, 6 weeks, 7 weeks, or 8 weeks.

The timing for administering the pharmaceutical composition according to the present invention to a patient or subject is not particularly limited, and examples thereof include immediately after onset of a disease, within n days (n represents an integer of 1 or more) from onset, immediately after diagnosis, within n days (n represents an integer of 1 or more) from diagnosis, before remission, during remission, after remission, before relapse, during relapse and after relapse.

The pharmaceutical composition according to the present invention can be preserved in a frozen state until immediately before use. The temperature for cryopreservation is preferably -30°C or lower, -40°C or lower, -50°C or lower, -80°C or lower, -90°C or lower, - 100°C or lower, -150°C or lower, -180°C or lower or -196°C (liquid nitrogen temperature) or lower. When the pharmaceutical composition according to the present invention is administered to a patient or subject, it can be used after being quickly thawed at 37°C.

The present invention will be specifically explained with reference to the Examples below; however, the present invention is not limited to the Examples.

Examples

<Comparative Example 1: Study on indices>

In the following Comparative Example 1 and Example 1, indices for obtaining a cell population comprising adherent stem cells exhibiting high immunosuppressive activity and/or proliferative property were investigated.

(Step 1: Collection of amnion)

A fetal membrane and a placenta, which are fetal appendages, were aseptically collected from a pregnant woman (donor A) of an elective cesarean section case after obtaining informed consent. The obtained fetal membrane and placenta were comprised in a sterile tray comprising physiological saline. An amnion was manually separated from the stump of the fetal membrane. The amnion was washed with a Hank's balanced salt solution (free of Ca and Mg) to remove attached blood and clots.

(Step 2: Enzymatic treatment of amnion and collection of amniotic MSCs)

The amnion obtained from donor A was immersed in a Hank's balanced salt solution (comprising Ca and Mg) comprising 240 PU/mL collagenase and 200 PU/mL dispase I and stirred while shaking at 37°C, for 90 minutes and at 50 rpm to enzymatically treat the amnion. The solution after enzymatic treatment was filtered through a nylon mesh having openings of 95 $\mu$m to remove undigested products of the amnion so as to collect a cell suspension comprising amniotic MSCs.

The obtained cell suspension was analyzed for the proportion of cells positive for the expression of CD90, which is one of surface antigens known as a typical positive marker of MSCs, using a flow cytometer. It was then confirmed that amniotic MSCs were able to be separated with high purity from the amnion.

The surface antigen analysis was carried out by using BD Accuri™ C6 Flow Cytometer from Becton, Dickinson and Company (BD) in the measurement conditions: analyzed cell number: 10,000 cells and flow rate setting: Slow (14 $\mu$L/min), In this measurement, FITC Mouse IgG1, $\kappa$ Isotype Control (BD/model number: 550616) was used as the antibody for isotype control and FITC Mouse Anti-Human CD90 (BD/model number: 555595) was used as the antibody for CD90 antigen.

(Step 3: Culture of amniotic MSCs)

A part of the cell population comprising amniotic MSCs obtained in the above section "Step 2: Enzymatic treatment of amnion and collection of amniotic MSCs" was seeded into a culture vessel, CellSTACK (registered trademark) (manufactured by Corning Incorporated) at a density of 6000 cells/cm$^2$ and adherent-cultured in $\alpha$MEM (Alpha Modification of Minimum Essential Medium Eagle) comprising 10% (final concentration) fetal bovine serum (FBS) (inactivated) and 10 ng/mL (final concentration) basic fibroblast growth factor (bFGF), until subconfluence. The cells adherent-cultured is referred to as the zero-th passage cell population.

The zero-th passage cell population was dissociated by using TrypLE Select (manufactured by Thermo Fisher Scientific Inc.) and a 1/5 amount of the cell population was seeded into CellSTACK (registered trademark) and subcultured. The subcultured cell population is referred to as the first-passage cell population.

The first-passage cell population that reached subconfluence was dissociated by using TrypLE Select, diluted with the medium and centrifugally collected. The collected cell population was suspended with RPMI1640. To this, the same amount of a CP-1 (registered trademark) solution (a solution prepared by mixing CP-1 (registered trademark) and 25 % human serum albumin in a ratio of 34: 16) (manufactured by Kyokuto Pharmaceutical Industrial Co., Ltd.) was added. The resultant mixture was gradually frozen up to -80°C and cryopreserved in liquid nitrogen. Thereafter, the cell population was thawed and seeded at a density of 15000 to 18000 cells/cm$^2$ in CellSTACK (registered trademark) and subcultured until subconfluence. The cell population subcultured is referred to as the second-passage cell population.

The second-passage cell population was dissociated by TrypLE Select and a 1/5 amount of the cell population was seeded into CellSTACK (registered trademark) and subcultured. The cell population subcultured is referred to as the third-passage cell population.

The third-passage cell population that reached subconfluence was dissociated by using TrypLE Select, diluted with the medium and centrifugally collected. The collected cell population was suspended with RPMI1640. To this, the same amount of a CP-1 (registered trademark) solution was added, gradually frozen up to -80°C. The resultant mixture was cryopreserved in liquid nitrogen. Thereafter, the cell population was thawed and seeded at a density of 6000 cells/cm$^2$ in CellSTACK (registered trademark) and subcultured until subconfluence. The cell population subcultured is referred to as the fourth-passage cell population.

The fourth-passage cell population was dissociated by TrypLE Select and a 1/5 amount of the cell population was seeded into CellSTACK (registered trademark) and subcultured. The cell population subcultured is referred to as the fifth-passage cell population.

The fifth-passage cell population that reached subconfluence was dissociated by using TrypLE Select, diluted with the medium and centrifugally collected. The collected cell population was suspended with RPMI1640. To this, the same amount of a CP-1 (registered trademark) solution was added. The resultant mixture was gradually frozen up to -80°C and thereafter cryopreserved in liquid nitrogen.

(Step 4: Surface antigen analysis)

Individual surface antigens (CD73 positive rate, CD90 positive rate, CD105 positive rate, CD45 positive rate and negative rate) of the fifth-passage cell population of Comparative Example 1 were checked by a flow cytometer.

The surface antigen analysis was carried out by use of BD Accuri™ C6 Flow Cytometer manufactured by Becton, Dickinson (BD) and Company (BD) in the measurement conditions: analysis cell number: 10,000 cells and flow rate setting: Slow (14 $\mu$L/min), In this measurement, FITC Mouse IgG1, $\kappa$ Isotype Control (BD/model number: 550616) was used as an isotype control antibody; FITC Mouse Anti-Human CD73 (BD/model number: 561254) as an antibody for CD73 antigen, FITC Mouse Anti-Human CD90 (BD/model number: 555595) as an antibody for CD90 antigen, Anti-Human Antibodies FITC Conjugate (BioLegend company/model number: 323203) as an antibody for CD105 antigen, and FITC Mouse Anti-Human CD45 (BD/model number: 555482) as an antibody for CD45 antigen.

As a result of the surface antigen analysis, in the fifth-passage cell population of Comparative Example 1, the CD73, CD90, and CD105 positive rates were all 90% or more (specifically, CD73: 100%, CD90: 100%, CD105: 98%); whereas the CD45 positive rate was less than 5% (negative rate: 95% or more) (specifically, CD45 positive rate: 0% (negative rate: 100%)). From the results, it was confirmed that the fifth-passage cell population of Comparative Example 1 is a cell population comprising adherent stem cells.

(Step 5: Gene expression analysis)

The fifth-passage cell population of Comparative Example 1 was subjected to analysis for expressions of PLIN2 gene, NEFM gene and SDHA gene using quantitative RT-PCR. The quantitative RT-PCR was carried out specifically by the following procedure A (comparison Ct method).

(Procedure A: Comparison Ct method)

(1) A cryopreserved cell population was thawed and centrifugally collected. The collected cell population was washed with phosphate buffer (PBS) and centrifugally collected.

(2) Total RNA was extracted from the cell population and purified by an RNA extraction kit (RNeasy Plus Mini kit (manufactured by QIAGEN)).

(3) Using the total RNA purified as a template and ReverTra Ace qPCR RT Master Mix with gDNA Remover (manufactured by Toyobo Co., Ltd.), cDNA was synthesized by a reverse transcription reaction.

(4) PCR was carried out using the synthesized cDNA as a template and KOD SYBR qPCR Mix (manufactured by Toyobo Co., Ltd.). The volume of a reaction solution was set at 20 μL. The PCR was carried out by use of StepOnePlus Real-Time PCR System (manufactured by Thermo Fisher Scientific Inc.) in the conditions: 98°C, 2 minutes hold, (98°C 10 seconds, 60°C 10 seconds, 68°C 30 seconds) 40 cycles.

(5) The relative expression level (X) of a measurement target gene to a comparative gene was calculated in accordance with $X = 2^{\{-(Y-Z)\}}$, where Y represents the Ct value of a measurement target gene and Z represents the Ct value of a comparative gene.

As a result, for the fifth-passage cell population of Comparative Example 1, the relative expression level of PLIN2 gene to the expression level of SDHA gene was 1.11 and the relative expression level of NEFM gene to the expression level of SDHA gene was 0.04. From the above, it was found that the fifth-passage cell population of Comparative Example 1 does not satisfy the following condition (b):

(b) The relative expression level of PLIN2 gene to the expression level of SDHA gene is 1.50 or more.

Note that, when the quantitative RT-PCR of the fifth-passage cell population of Comparative Example 1 was carried out by the following procedure B (calibration method), the relative expression level of PLIN2 gene to the expression level of SDHA gene was 0.27 and the relative expression level of NEFM gene to the expression level of SDHA gene was 1.51.

(Procedure B: Calibration method)

(1) A cryopreserved cell population was thawed and centrifugally collected. The collected cell population was washed with phosphate buffer (PBS) and centrifugally collected.

(2) Total RNA was extracted from the cell population and purified by RNA extraction kit (RNeasy Plus Mini kit (manufactured by QIAGEN)).

(3) Using the purified total RNA as a template and ReverTra Ace qPCR RT Master Mix with gDNA Remover (manufactured by Toyobo Co., Ltd.), cDNA was synthesized by reverse transcription.

(4) Using synthesized cDNA as a template and Power SYBR Green PCR Master Mix (manufactured by Thermo Fisher Scientific Inc.), a PCR was carried out. The volume of a reaction solution was set at 20 μL. The PCR was carried out by use of StepOnePlus Real-Time PCR System (manufactured by Thermo Fisher Scientific Inc.) in the conditions: 50°C, 2 minutes hold, 95°C, 2 minutes hold (95°C 3 seconds, 60°C 30 seconds) 60 cycles. Note that, as a sample for preparing a calibration curve, synthesized cDNA was subjected to double-fold serial dilution, one to five times, and put in use.

(5) The expression levels of the comparative gene and measurement target genes displayed by StepOnePlus Real-Time PCR System (manufactured by Thermo Fisher Scientific Inc.) were recorded.

(6) The relative expression level (W) of the measurement target gene to the comparative gene was calculated in accordance with $W = \alpha/\beta$, where $\alpha$ represents the expression level of a measurement target gene and $\beta$ represents the expression level of a comparative gene.

(Step 6: Evaluation of TNFAIP6 gene expression level)

The expression level of TNFAIP6 gene of the fifth-passage cell population of Comparative Example 1 was evaluated by a microarray. As a result, for the fifth-passage cell population of Comparative Example 1, the relative expression level of TNFAIP6 gene to the expression level of SDHA gene was 0.41.

(Step 7: Evaluation of the proliferation inhibition rate of hPBMCs)

The immunosuppressive activity of the fifth-passage cell population of Comparative Example 1 was evaluated by the mixed lymphocyte reaction test (MLR test). As a result, the fifth-passage cell population of Comparative Example 1 had a proliferation inhibition rate of hPBMCs of 42.0%.

(Step 8: Evaluation of specific growth rate)

The specific growth rate of the fifth-passage cell populations of Comparative Example 1 and Example 1 were evaluated. As a result, the specific growth rate of cell population of Comparative Example 1 was 0.24 (1/day).

<Example 1: Study on indices>

(Step 1: Collection of amnion)

An amnion was obtained in the same manner as in Step 1 of Comparative Example 1 except that a fetal membrane and a placenta, which are fetal appendages, were aseptically collected from the same donor as the donor of Comparative Example 1 (donor A) and a donor different from the donor of Comparative Example 1 (donor B).

(Step 2: Enzymatic treatment of amnion and collection of amniotic MSCs)

A cell suspension comprising amniotic MSCs was collected by the same method as in Step 2 of Comparative Example 1. The obtained cell suspension was analyzed in the same manner as in Comparative Example 1 for the proportion of cells positive for the expression of CD90. It was confirmed that amniotic MSCs were able to be separated with high purity from the amnion.

(Step 3: Culture of amniotic MSCs)

The fifth-passage cell population was obtained in the same manner as in Step 3 of Comparative Example 1 except that α MEM comprising a 5% human platelet lysate (hPL) was used in place of α MEM comprising 10% FBS and 10 ng/mL bFGF.

(Step 4: Surface antigen analysis)

Surface antigen analysis of the fifth-passage cell population of Example 1 was carried out in the same manner as in Step 4 of Comparative Example 1. As a result, in the fifth-passage cell population derived from donor A of Example 1, the CD73, 90 and 105-positive rates were all 90% or more (specifically, CD73: 98%, CD90: 100%, CD105: 100%); whereas, the CD45 positive rate was less than 5% (negative rate: 95% or more) (specifically, CD45 positive rate: 0% (negative rate: 100%)). In the fifth-passage cell population derived from donor B of Example 1, the CD73, 90 and 105 positive rates were all 90% or more (specifically, CD73: 100%, CD90: 99%, CD105: 98%); whereas, the CD45 positive rate was less than 5% (negative rate: 95% or more) (specifically, CD45 positive rate: 0% (negative rate: 100%)). From the results, it was confirmed that the fifth-passage cell population of Example 1 is a cell population comprising adherent stem cells.

(Step 5: Gene expression analysis)

Gene expression analysis of the fifth-passage cell population of Example 1 was carried out in accordance with procedure A (comparison Ct method) described in Step 5 of Comparative Example 1. The relative expression levels of individual genes are shown in Table 1.

[Table 1]

Table 1. Relative expression level of genes

|  | PLIN2 /SDHA | NEFM /SDHA | PLIN2 /NEFM |
|---|---|---|---|
| Fifth-passage cell population derived from donor A of Example 1 | 1.95 | 0.0006 | 3051 |
| Fifth-passage cell population derived from donor B of Example 1 | 5.32 | 0.017 | 315 |
| Fifth-passage cell population of Comparative Example 1 | 1.11 | 0.04 | 26 |

As shown in Table 1, for the fifth-passage cell population derived from donor A of Example 1, the relative expression level of PLIN2 gene to the expression level of SDHA gene was 1.95; the relative expression level of NEFM gene to the expression level of SDHA gene was0.0006; and the relative expression level of PLIN2 gene to the expression level of NEFM gene was 3051. For the fifth-passage cell population derived from donor B of Example 1, the relative expression level of PLIN2 gene to the expression level of SDHA gene was 5.32; the relative expression level of NEFM gene to the expression level of SDHA gene was 0.017; and the relative expression level of PLIN2 gene to the expression level of NEFM gene was 315.

Thus, it was found that the fifth-passage cell populations of Example 1 satisfy the following conditions (a) and (b):

(a) the cell population is positive for expressions of PLIN2 gene and NEFM gene,
(b) the cell population satisfies the relative expression level of PLIN2 gene to the expression level of SDHA gene of 1.50 or more.

Note that, the gene expression analysis was carried out in accordance with procedure B (calibration method) described in Step 5 of Comparative Example 1. As a result, the relative expression levels of individual genes of the fifth-passage cell population of Example 1 were as shown in Table 2.

[Table 2]

Table 2. Relative expression levels of genes to the expression level of SDHA gene

|  | PLIN2 gene | NEFM gene |
|---|---|---|
| Fifth-passage cell population derived from donor A of Example 1 | 0.96 | 0.03 |
| Fifth-passage cell population derived from donor B of Example 1 | 1.25 | 0.26 |
| Fifth-passage cell population of Comparative Example 1 | 0.27 | 1.51 |

(Step 6: Evaluation of TNFAIP6 gene expression level)

The expression level of TNFAIP6 gene of the fifth-passage cell population derived from donor A of Example 1 was evaluated in the same manner as in Step 6 of Comparative Example 1. As a result, for the fifth-passage cell population derived from donor A of Example 1, the relative expression level of TNFAIP6 gene to the expression level of SDHA gene was 1.96. From the above, it was suggested that the fifth-passage cell population derived from donor A of Example 1 highly expresses TNFAIP6 gene, which is a gene encoding a cytokine involved in immunosuppression, and exhibits high immunosuppressive activity.

(Step 7: Evaluation of proliferation inhibition rate of hPBMCs)

The immunosuppressive activity of the fifth-passage cell population derived from donor A of Example 1 was evaluated in the same manner as in Step 7 of Comparative Example 1. As a result, it was found that for the fifth-passage cell population derived from donor A of Example 1, the proliferation inhibition rate of hPBMCs was 54.4%. From the above, it was found that the fifth-passage cell population derived from donor A of Example 1 significantly inhibits proliferation of hPBMCs activated by stimulation of PHA and exhibits high immunosuppressive activity.

Thus, it was found that the cell population having cell characteristics of the above (a) and (b), exhibits high immunosuppressive activity. It was suggested that the conditions of the above (a) and (b) are effective as indices for obtaining a cell population comprising adherent stem cells exhibiting high immunosuppressive activity.

(Step 8: Evaluation of specific growth rate)

The specific growth rate of the fifth-passage cell population of Example 1 was evaluated in the same manner as in Step 8 of Comparative Example 1. The measurement results of the specific growth rate are shown in Table 3.

[Table 3]

Table 3. Specific growth rate of cell populations of Comparative Example 1 and Example 1

|  | Specific growth rate (1/day) |
|---|---|
| Fifth-passage cell population derived from donor A of Example 1 | 0.55 |
| Fifth-passage cell population derived from donor B of Example 1 | 0.87 |
| Fifth-passage cell population of Comparative Example 1 | 0.24 |

As shown in Table 3, the specific growth rate of the cell population derived from donor A of Example 1 was 0.55 (1/day); whereas, the specific growth rate of the cell population derived from donor B of Example 1 was 0.87 (1/day). From the above, it was found that the fifth-passage cell population of Example 1 exhibits high proliferative property.

Thus, it was found that the cell population having cell characteristics of the above (a) and (b) exhibits high proliferative property. It was suggested that the conditions of the above (a) and (b) are effective as indices for obtaining a cell population comprising adherent stem cells exhibiting high proliferative property.

From the above results, it was found that the cell population having cell characteristics of the above (a) and (b) exhibits high proliferative property in addition to high immunosuppressive activity. It was suggested that the conditions of the above (a) and (b) are effective as indices for obtaining a cell population comprising adherent stem cells exhibiting high immunosuppressive activity and/or high proliferative property. In other words, according to the present invention, a cell population comprising adherent stem cells exhibiting high immunosuppressive activity and/or high proliferative property can be obtained by using conditions of the above (a) and (b) as indices, with the result that cell culture period can be reduced and manufacturing cost can be lowered.

<Example 2: Study on immunosuppressive activity using hPBMC-induced GVHD mice>

In Example 2, whether or not a cell population having the following cell characteristic (a) and (b) exhibits immunosuppressive activity in animal experimentation was examined.

(a) the cell population is positive for expressions of PLIN2 gene and NEFM gene,
(b) the cell population satisfies the relative expression level of PLIN2 gene to the expression level of SDHA gene of 1.50 or more.

(Step 1: Collection of amnion)

Amnion was obtained in the same manner as in Step 1 of Comparative Example 1 except that egg membrane and placenta as a fetal appendage, were aseptically collected from a donor (donor E) different from the donors in Comparative Example 1 and Example 1.

(Step 2: Enzymatic treatment of amnion and collection of amniotic MSCs)

A cell suspension comprising amniotic MSCs was collected in the same manner as in Step 2 of Comparative Example 1.

(Step3: Culture of amniotic MSCs)

A cell population was obtained in the same manner as in Step 3 of Example 1.

(Step 4: Gene expression analysis)

In the same procedure A (comparison Ct method) as in Step 5 of Comparative Example 1, gene expression analysis of the fifth-passage cell population derived from donor E was carried out. The relative expression levels are shown in Table 4.

[Table 4]

Table 4. Relative expression levels of genes

| | PLIN2 /SDHA | NEFM /SDHA | PLIN2 /NEFM |
|---|---|---|---|
| Fifth-passage cell population derived from donor E | 7.94 | 0.0004 | 20343 |

As shown in Table 4, for the fifth-passage cell population derived from donor E, the relative expression level of PLIN2 gene to the expression level of SDHA gene was 7.94; the relative expression level of NEFM gene to the expression level of SDHA gene was 0.0004; and the relative expression level of PLIN2 gene to the expression level of NEFM gene was 20343.

Thus, it was found that the fifth-passage cell population derived from donor E satisfies the following conditions (a) and (b).

(a) the cell population is positive for expressions of PLIN2 gene and NEFM gene,
(b) the cell population satisfies the relative expression level of PLIN2 gene to the expression level of SDHA gene of 1.50 or more.

Also, as shown in Table 4, for the fifth-passage cell population derived from donor E, the relative expression level of NEFM gene to the expression level of SDHA gene was less than 0.02.

Further, as shown in Table 4, for the fifth-passage cell population derived from donor E, the relative expression level of PLIN2 gene to the expression level of NEFM gene was 250 or more.

(Step 5: Evaluation of proliferation inhibition rate of hPBMCs)

The immunosuppressive activity of the fifth-passage cell population derived from donor E was evaluated by the mixed lymphocyte reaction test (MLR test). As a result, for the cell population, the proliferation inhibition rate of hPBMCs was 53.2%. From the above, it was found that the fifth-passage cell population derived from donor A of Example 1 inhibits proliferation of hPBMCs activated by stimulation of PHA and exhibits immunosuppressive activity.

(Step 6: Evaluation of immunosuppressive activity by using hPBMC-induced GVHD mice)

In mice (hPBMC-induced GVHD mice) having a graft-versus-host disease (GVHD) induced by human peripheral blood mononuclear cells (hPBMCs), the immunosuppressive activity of the fifth-passage cell population derived from donor E was evaluated.

The hPBMC-induced GVHD mice were prepared with reference to the description of Ito et al., Transplantation 2009, 87: 1654-1658, Ito et al., Am J Transplant. 2017 May; 17 (5): 1216-1228. Specifically, male mice of NOD/Shi-scid, IL-2RyKO (NOG) lineage, 7-8 weeks old were administered with hPBMCs ($3.0 \times 10^6$ cells/0.2 mL/Head) at a rate of 1.2 mL/minute through the tail vein. The day on which hPBMCs was administered was defined as Day 0.

The fifth-passage cell population derived from donor E and cryopreserved was thawed, diluted with physiological saline and centrifuged. A part of the supernatant was removed to prepare a resuspension of the cell population so as to compris $1.6 \times 10^6$ cells/mL. To the hPBMC-induced GVHD mice prepared using the hPBMCs, the above cell population was administered at a rate of 1.2 mL/minute through the tail vein. The dose was set at $8.0 \times 10^6$ cells/5mL/kg body weight. The number of doses was 3. In the first-half administration group, administration was made on Day 7, 14 and 21. In the second-half administration group, administration was made on Day 21, 28 and 35. Note that, hPBMC-induced GVHD mice not administered with the cell population were employed as a control group.

The viability of the above groups was evaluated. Survival analysis (Log-RANK test) was used for the viability evaluation. In the evaluation of the viability, a case having a risk factor of less than 5% ($p < 0.05$) was determined as being significantly different.

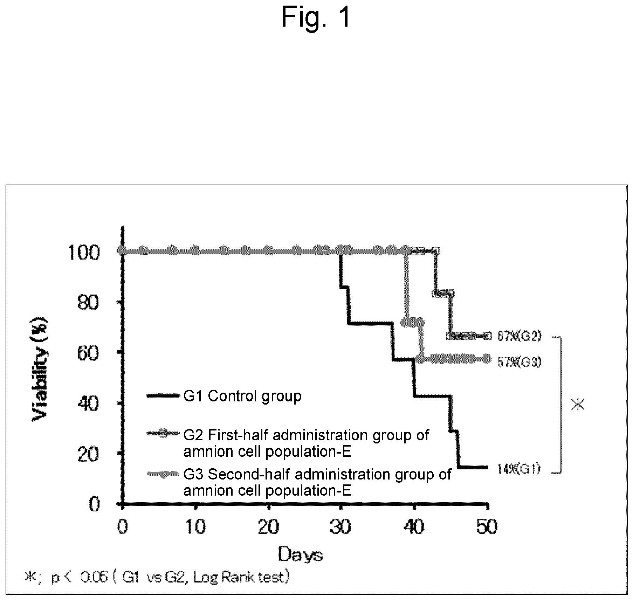

The results of the viability are shown in Figure 1.

In the results, the viability of the hPBMC-induced GVHD mice in the first-half administration group was significantly improved compared to that in the control group; and the viability of the hPBMC-induced GVHD mice in the second-half administration group was likely improved compared to that in the control group. Also, weight loss of the hPBMC-induced GVHD mice in the first-half administration group was likely suppressed compared to that in the control group. From the above, it was suggested that the fifth-passage cell population derived from donor E improves GVHD in the hPBMC-induced GVHD mice.

Thus, it was revealed that the cell population having cell characteristics of the above (a) and (b) exhibits immunosuppressive activity not only in the mixed lymphocyte reaction test (MLR test) but also in the hPBMC-induced GVHD mice. It was suggested that the conditions of the above (a) and (b) are effective as indices for obtaining a cell population comprising adherent stem cells exhibiting high immunosuppressive activity.

(Step 7: Evaluation of specific growth rate)

Specific growth rate of the fifth-passage cell population derived from donor E was evaluated. As a result, the specific growth rate of the fifth-passage cell population derived from donor E was 0.44 (1/day).
Thus, it was revealed that the cell population having cell characteristics of the above (a) and (b) shows high proliferation property. It was suggested that the conditions of the above (a) and (b) are effective as indices for obtaining a cell population comprising adherent stem cells exhibiting high proliferation property.
The above results of Example 2 are collectively shown in Table 5.

[Table 5]

Table 5. Summary of Example 2

|  | Cell characteristics (a), (b) | hPBMCs proliferation inhibition rate | hPBMC-induced GVHD mouse | Specific growth rate (1/day) |
|---|---|---|---|---|
| Cell population derived from donor E | Satisfied | 53.2 | GCDH was improved | 0.44 |

From Table 5, it was found that the cell population having cell characteristics of the above (a) and (b) exhibits high proliferation property in addition to high immunosuppressive activity in the mixed lymphocyte reaction test (MLR test) and in the hPBMC-induced GVHD mice. It was suggested that the conditions of the above (a) and (b) are effective as indices for obtaining a cell population comprising adherent stem cells exhibiting high immunosuppressive activity and/or high proliferative property. In short, according to the present invention, a cell population comprising adherent stem cells exhibiting high immunosuppressive activity and/or high proliferative property can be obtained by using the condition of the above (a) and (b) as indices, with the result that cell culture period can be reduced and manufacturing cost can be lowered.

<Example 3: Manufacture of pharmaceutical composition>

A part of the fifth-passage cell population of Example 1 is subjected to preparation of a pharmaceutical composition. A pharmaceutical composition (cell preparation) comprising $2.0 \times 10^8$ adherent stem cells, 6.8 mL of a CP-1 (registered trademark) solution, 3.2 mL of a 25 % human serum albumin solution and a 10 mL of RPMI1640 culture medium is prepared. The pharmaceutical composition is enclosed in a freezing bag and stored in a frozen state. Note that the pharmaceutical composition can be thawed upon use and applied to a patient or subject.

<Example 4: Preparation of amnion-derived adherent stem cells>

The second-passage cell population was obtained in the same manner as in Steps 1 to 3 of Example 1 except that the fetal membrane was collected from two donors (donor F, G), who are different from those of Comparative Example 1, Example 1 and Example 2.

<Comparative Example 2: Preparation of bone marrow-derived adherent stem cells>

The whole amount of a commercially available bone marrow fluid (Company: AllCells/product number: ABM003) was seeded and subjected to primary culture performed in $\alpha$ MEM (Alpha Modification of Minimum Essential Medium Eagle) comprising 10% (final concentration) fetal bovine serum (FBS) (inactivated). A part of the obtained zero-th passage cell population comprising the bone marrow-derived adherent stem cells was seeded at a density of 6000 cells/cm² in CellSTACK (registered trademark) (manufactured by Corning Incorporated) and subcultured in $\alpha$ MEM comprising 10% (final concentration) FBS (inactivated). The obtained cell population comprising bone marrow-derived adherent stem cells was cryopreserved in a cell storage solution comprising CP-1 (registered trademark) solution.

<Example 5: Surface antigen analysis>

(Surface antigen analysis)

Cell populations obtained in Example 4, Comparative Example 1 and Comparative Example 2 were analyzed for surface antigen (CD73, CD90, CD105, CD45, CD326) by flow cytometry dot-plot analysis. The flow cytometry dot-plot analysis was carried out in accordance with the procedures (1) to (8).
The ratios of cells positive for individual surface antigens are shown in Table 6.

[Table 6]

Table 6. The ratios of cells positive for surface antigens

|  | CD73 | CD90 | CD105 | CD45 | CD326 |
|---|---|---|---|---|---|
| Amnion cell population-F (Example 4) | 99% | 99% | 91% | 0% | 0% |
| Amnion cell population-G (Example 4) | 99% | 93% | 93% | 0% | 0% |
| Amnion cell population-A (Comparative Example 1) | 99% | 98% | 98% | 0% | 0% |
| Bone marrow cell population (Comparative Example 2) | 97% | 98% | 97% | 0% | 0% |

As shown in Table 6, in the cell populations, positive rates of CD73, CD90, and CD105 were all 90% or more; whereas the positive rates of CD45 and CD326 were less than 5% (negative rate: 95% or more).

<Example 6: Evaluation of gene expression level>

In accordance with the procedure A (comparison Ct method) described in Step 5 of Comparative Example 1, gene expression analysis of cell populations obtained in Example 4, Comparative Example 1 and Comparative Example 2 was carried out. The relative expression levels of individual genes are shown in Table 7.

[Table 7]

Table 7. Relative expression level of genes

|  | PLIN2 /SDHA | NEFM /SDHA | PLIN2 /NEFM |
|---|---|---|---|
| Amnion cell population-F (Example4) | 2.02 | 0.001 | 1535 |
| Amnion cell population-G (Example4) | 2.12 | 0.002 | 1047 |
| Amnion cell population-A (Comparative Example1) | 1.11 | 0.04 | 26 |
| Bone marrow cell population (Comparative Example2) | 1.31 | 0.006 | 223 |

As shown in Table 7, in amnion cell population-F, -G, and -A and bone marrow cell population, expressions of PLIN2 gene and NEFM gene were positive. Also, in either one of amnion cell population-F and -G, the relative expression level of PLIN2 gene to SDHA gene satisfied 1.50 or more (amnion cell population-F: 2.02, amnion cell population-G: 2.12). In contrast, in amnion cell population-A and bone marrow cell population, the relative expression level of PLIN2 gene to SDHA gene satisfied less than 1.50 (amnion cell population-A: 1.11, bone marrow cell population: 1.31). From the above, it was found that amnion cell population-F and -G satisfy the following conditions (a) and (b). In contrast, it was found that neither amnion cell population-A nor bone marrow cell population satisfies the above condition (b).

(a) the cell population is positive for expressions of PLIN2 gene and NEFM gene,
(b) the cell population satisfies the relative expression level of PLIN2 gene to the expression level of SDHA gene of 1.50 or more.

As shown in Table 7, for amnion cell population-F, -G and bone marrow cell population, the relative expression level of NEFM gene to SDHA gene was less than 0.02. For amnion cell population-A, the relative expression level of NEFM gene to SDHA gene was 0.02 or more.

As shown in Table 7, for amnion cell population-F and -G, the relative expression level of PLIN2 gene to the expression level of NEFM gene was 250 or more. For amnion cell population-A and bone marrow cell population, the relative expression level of PLIN2 gene to the expression level of NEFM gene was less than 250.

<Example 7: Evaluation of immunosuppressive activity using hPBMC-induced GVHD mice>

In mice (hPBMC-induced GVHD mice) having a graft-versus-host disease (GVHD) induced by human peripheral blood mononuclear cells (hPBMCs), the immunosuppressive activity of cell populations (amnion cell population-F, A and bone marrow cell population) obtained in Example 4, Comparative Example 1 and Comparative Example 2 was evaluated.

Animal experiment was carried out in the same manner as in Step 6 of Example 2 except that the number of doses was changed from 3 to 4 (administered on Day 7, 14, 21 and 28). Note that, a group of hPBMC-induced GVHD mice not administered with the above cell populations were used as a control.

A change in viability of each of the above groups over time was evaluated. The viability was evaluated by survival analysis (Log-RANK test). In the change of viability over time, a case having a risk factor of less than 5% (p < 0.05) was determined as being significantly different.

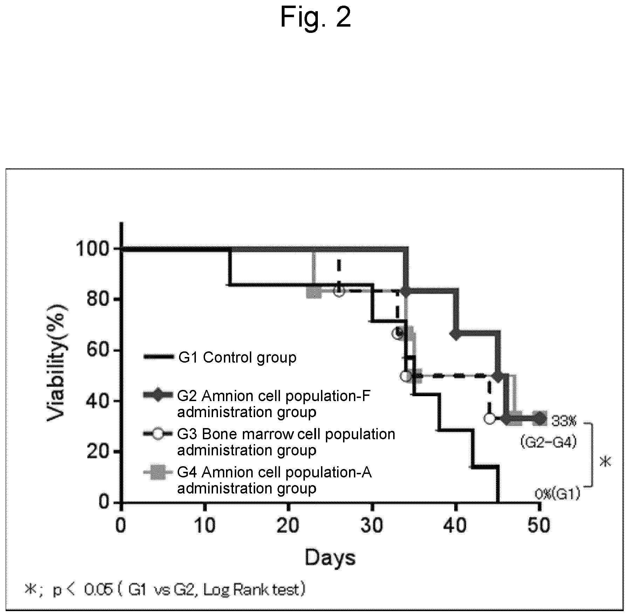

The results of the viability are shown in Figure 2.

As shown in Figure 2, the viability of the hPBMC-induced GVHD mice was significantly improved in the amnion cell population-F administration group compared to that in the control group; and the viability of the hPBMC-induced GVHD mice was likely improved in the amnion cell population-A administration group, and the bone marrow cell population administration group, compared to that in the control group. Also, in the amnion cell population-F administration group, weight loss of the hPBMC-induced GVHD mice was likely suppressed, compared to that in the control group. From the above, it was revealed that amnion cell population-F has high GVHD improving effect in the hPBMC-induced GVHD mice, compared to that in other cell populations (amnion cell population-A, bone marrow cell population). In other words, the amnion cell population-F exhibits higher immunity compared to that in other cell populations. Accordingly, it was suggested that the cell population satisfying the conditions of the above (a) and (b) exhibits high immunosuppressive activity. From the above, it can be said that the conditions of the above (a) and (b) are effective as indices for obtaining cell population comprising adherent stem cells exhibiting high immunosuppressive activity.

<Example 8: Quality evaluation of biological samples collected from different donors>

In Example 8, whether or not the quality of a donor itself and a biological sample collected from the donor can be evaluated by using the conditions of the following (a) and (b) as indices, was examined.

(a) the cell population is positive for expressions of PLIN2 gene and NEFM gene,
(b) the cell population satisfies the relative expression level of PLIN2 gene to the expression level of SDHA gene of 1.50 or more.

(Step 1: Collection of amnion)

The amnion was obtained by the same manner as in Step 1 of Comparative Example 1 except that the fetal membrane and placenta as a fetal appendage, were aseptically collected from two donors (donors C and D), who are different from those of Comparative Example 1, Example 1, Example 2 and Example 4.

(Step 2: Enzymatic treatment of amnion and collection of amniotic MSCs)

A cell suspension comprising amniotic MSCs was collected in the same manner as in Step 2 of Example 1.

(Step 3: Culture of amniotic MSC)

A zero-th passage cell population was obtained in the same manner as in Step 3 of Example 1.

(Step 4: Gene expression analysis)

Gene expression analysis of the zero-th passage cell populations derived from individual donors (donor C, D) was carried out in accordance with procedure A (comparison Ct method) described in Step 5 of Comparative Example 1. As a result, the zero-th passage cell population derived from donor C satisfied the conditions of neither the above (a) nor (b). In contrast, the zero-th passage cell population derived from donor D satisfied the conditions of the above (a) and (b).

(Step 5: Evaluation of specific growth rate)

The specific growth rate of the zero-th passage cell population derived from each of the donors (donor C, D) was evaluated. The measurement results of specific growth rate are shown in Table 8.

[Table 8]

Table 8. Specific growth rate of zero-th passage cell populations derived from donors

|  | Specific growth rate (1/day) |
|---|---|
| Zero-th passage cell population derived from donor C | 0.18 |
| Zero-th passage cell population derived from donor D | 0.46 |

As shown in Table 8, the specific growth rate of the zero-th passage cell population derived from donor C was 0.18 (1/day) and the specific growth rate of the zero-th passage cell population derived from donor D was 0.46 (1/day). From the above, it was found that the zero-th passage cell population derived from donor D has high proliferation property.

From the results, it was shown that if amniotic MSCs were collected and cultured in the same method from the amnion, proliferative property of the cell population significantly differs depending on the difference in donor and suggested that if the conditions of the above (a) and (b) are investigated, the quality of the donor itself and a biological sample collected from the donor can be evaluated. In short, according to the present invention, the quality of a donor itself and a biological sample collected from the donor can be evaluated by using the conditions of the above (a) and (b) as indices, a biological sample high in the content of adherent stem cells having high proliferative property can be selected in advance (donor screening). In this manner, unnecessary cell culture can be omitted or cell culture period can be reduced, with the result that manufacturing cost can be lowered.

<Example 9: Monitoring in culture step>

In Example 9, whether or not the immunosuppressive activity and/or proliferative property of adherent stem cells can be monitored by using the conditions of the following (a) and (b) as indices in the culture step was examined.

　　(a) the cell population is positive for expressions of PLIN2 gene and NEFM gene,
　　(b) the cell population satisfies the relative expression level of PLIN2 gene to the expression level of SDHA gene of 1.50 or more.

(Step 3: Culture of amniotic MSC)

Since the zero-th passage cell population derived from donor D obtained in Step 3 of Example 8 satisfied the conditions of the above (a) and (b), the cell population was subjected to the following passage culture. The cell population was subcultured in the same manner as in Step 3 of Example 1 to obtain first to fifth-passage cell populations. Whether or not the conditions of the above (a) and (b) are satisfied was checked in every subculture. Since the zero-th passage cell population derived from donor C obtained in Step 3 of Example 8 satisfied the conditions of neither the above (a) nor (b), the cell population was not subjected to the following passage culture.

(Step 4: Gene expression analysis)

In the procedure A (comparison Ct method) described in Step 5 of Comparative Example 1, gene expression analysis of the first to fifth-passage cell populations derived from donor D was carried out. As a result, each of the cell populations satisfied the conditions of the above (a) and (b).

(Step 5: Evaluation of specific growth rate)

Specific growth rate was evaluated for the first to fifth-passage cell populations derived from donor D. The measurement results of specific growth rates of individual cell populations are shown in Table 9.

[Table 9]

Table 9. Specific growth rate of cell populations derived from donor D

|  | Specific growth rate (1/day) |
| --- | --- |
| First passage cell population derived from donor D | 0.64 |
| Second passage cell population derived from donor D | 0.68 |
| Third passage cell population derived from donor D | 0.41 |
| Fourth passage cell population derived from donor D | 0.56 |
| Fifth passage cell population derived from donor D | 0.40 |

As shown in Table 9, specific growth rates of the first to fifth-passage cell populations derived from donor D were 0.64 (1/day), 0.68 (1/day), 0.41 (1/day), 0.56 (1/day) and 0.40 (1/day), respectively. It was found that all of the cell populations exhibit high proliferation property even after subculture.

(Step 6: Evaluation of expression level of TNFAIP6 gene)

The expression levels of TNFAIP6 gene in the first to fifth-passage cell populations derived from donor D can be evaluated. Each of the cell populations expresses TNFAIP6 gene and has immunosuppressive activity.

From the above, the immunosuppressive activity and/or proliferative property of adherent stem cells can be monitored by using the conditions of the above (a) and (b) as indices in the culture step. In short, according to the present invention, changes of the immunosuppressive activity and/or proliferative property of adherent stem cells can be immediately obtained and predicted by measuring the indices (preferably, measuring over time). Owing to this, the period of cell culture can be reduced and manufacturing cost can be lowered.

**Claims**

1. A production method of a cell population comprising adherent stem cells, the method comprising obtaining a cell population having the following cell characteristics (a) and (b):

(a) the cell population is positive for expressions of PLIN2 gene and NEFM gene,
(b) the cell population satisfies the relative expression level of PLIN2 gene to the expression level of SDHA gene of 1.50 or more.

2. The production method according to claim 1, wherein the cell population satisfies the relative expression level of NEFM gene to the expression level of SDHA gene of less than 0.02.

3. The production method according to claim 1 or 2, wherein the cell population satisfies the relative expression level of PLIN2 gene to the expression level of NEFM gene of 250 or more.

4. A cell population comprising adherent stem cells and having the following cell characteristics (a) and (b):

(a) the cell population is positive for expressions of PLIN2 gene and NEFM gene,
(b) the cell population satisfies the relative expression level of PLIN2 gene to the expression level of SDHA gene of 1.50 or more.

5. The cell population according to claim 4, wherein the cell population satisfies the relative expression level of NEFM gene to the expression level of SDHA gene of less than 0.02.

6. The cell population according to claim 4 or 5, wherein the cell population satisfies the relative expression level of PLIN2 gene to the expression level of NEFM gene of 250 or more.

7. The cell population according to any one of claims 4 to 6, wherein the adherent stem cells are derived from a fetal appendage.

8. A pharmaceutical composition comprising the cell population according to any one of claims 4 to 7 and a pharmaceutically acceptable vehicle.

9. The pharmaceutical composition comprising the cell population according to any one of claims 4 to 7 and additional cells that can be administered.

10. The pharmaceutical composition according to claim 8 or 9, wherein a single dose of the adherent stem cells to a human is $1 \times 10^{12}$ cells/kg body weight or less.

11. The pharmaceutical composition according to any one of claims 8 to 10, wherein the pharmaceutical composition is an injectable preparation.

12. The pharmaceutical composition according to any one of claims 8 to 11, wherein the pharmaceutical composition is a transplant preparation of a cell aggregate or sheet-like structure.

13. The pharmaceutical composition according to any one of claims 8 to 12, being a therapeutic agent for an immune-related disease.

# Fig. 1

Fig. 2

## INTERNATIONAL SEARCH REPORT

International application No.

PCT/JP2018/048539

| A. CLASSIFICATION OF SUBJECT MATTER |
|---|
| Int.Cl. See extra sheet |

According to International Patent Classification (IPC) or to both national classification and IPC

| B. FIELDS SEARCHED |
|---|
| Minimum documentation searched (classification system followed by classification symbols) |
| Int.Cl. C12N1/00-7/08, C12Q1/00-3/00 |

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)
CAplus/DWPI/MEDLINE/EMBASE/BIOSIS (STN)

## C. DOCUMENTS CONSIDERED TO BE RELEVANT

| Category* | Citation of document, with indication, where appropriate, of the relevant passages | Relevant to claim No. |
|---|---|---|
| Y | JP 2015-61520 A (NATIONAL CEREBRAL AND CARDIOVASCULAR CENTER) 02 April 2015, paragraphs [0003], [0010], [0013], [0045], [0050], [0051], [0053]-[0060], [0095], [0103] & US 2016/0228474 A1 paragraphs [0003], [0020], [0023], [0116], [0121], [0122], [0124]-[0131], [0169], [0179] & WO 2015/025810 A1 & EP 3037523 A1 & CN 105765060 A | 1-13 |

☒ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 26 March 2019 (26.03.2019) | 02 April 2019 (02.04.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

# EP 3 733 838 A1

## INTERNATIONAL SEARCH REPORT

International application No.

PCT/JP2018/048539

C (Continuation). DOCUMENTS CONSIDERED TO BE RELEVANT

| Category* | Citation of document, with indication, where appropriate, of the relevant passages | Relevant to claim No. |
|---|---|---|
| Y | CHEVALLIER, Nathalie et al., "Osteoblastic differentiation of human mesenchymal stem cells with platelet lysate", Biomaterials, 2010, vol. 31, no. 2, pp. 270-278, 2.2 Bone marrow cell cultures, 3.1 Effect pf PL on MSC growth, fig. 1 | 1-13 |
| A | TONDREAU, Tatiana et al., "Gene expression pattern of functional neuronal cells derived from human bone marrow mesenchymal stromal cells", BMC Genomics, 2008, vol. 9, no. 166, pp. 1-11 | 1-13 |
| A | NIMURA, Satomi et al., "Olanzapine promotes the accumulation of lipid droplets and the expression of multiple perilipins in human adipocytes", Biochemical and Biophysical Research Communications, 2015, vol. 467, no. 4, pp. 906-912 | 1-13 |
| A | MAGUIRE, Colin T. et al., "Genome-wide analysis reveals the unique stem cell identity of human amniocytes", PLOS ONE, 2013, vol. 8, no. 1, e53372:p.1-16 | 1-13 |
| A | 辻紘子, 目で見る生殖と再生, HORMONE FRONTIER IN GYNECOLOGY, 2011, vol. 18, no. 4, pp. 4-8, non-official translation (TSUJI, Hiroko, "Visual reproduction and regeneration") | 1-13 |
| A | 梅川孝 ほか, 胎児付属物、特に羊膜を用いた再生医療の現状と展望, HORMONE FRONTIER IN GYNECOLOGY, 2016, vol. 23, no. 2, pp. 21-25, non-official translation (UMEKAWA, Takashi et al., "Recent status and the prospect of regenerative medicine using fetal appendages, especially amniotic membrane") | 1-13 |

Form PCT/ISA/210 (continuation of second sheet) (January 2015)

| INTERNATIONAL SEARCH REPORT | International application No. |
| --- | --- |
| | PCT/JP2018/048539 |

CLASSIFICATION OF SUBJECT MATTER
C12N5/0775(2010.01)i,     A61K35/28(2015.01)i,     A61K35/50(2015.01)i,
A61K35/51(2015.01)i,     A61P1/04(2006.01)i,     A61P3/10(2006.01)i,
A61P17/06(2006.01)i,     A61P19/02(2006.01)i,     A61P25/00(2006.01)i,
A61P29/00(2006.01)i,     A61P37/02(2006.01)i,     A61P37/06(2006.01)i,
C12N15/09(2006.01)n, C12Q1/6844(2018.01)n

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015061520 A
- WO 2017073656 A
- WO 2013077428 A
- WO 2011043136 A
- JP 2013256510 A

- WO 2017126549 A
- WO 2006080434 A
- JP 2016052272 A
- JP 2017529362 A


**Non-patent literature cited in the description**

- **ONKEN JE et al.** *American College of Gastroenterology Conference,* 2006
- **GARCIA-OLMO D et al.** *Dis Colon Rectum,* 2005, vol. 48, 1416-23

- **ITO et al.** *Transplantation,* 2009, vol. 87, 1654-1658
- **ITO et al.** *Am J Transplant.,* May 2017, vol. 17 (5), 1216-1228